(19) Europäisches Patentamt

European Patent Office

Office européen des brevets

(11) **EP 4 583 018 A1**

(12) **EUROPEAN PATENT APPLICATION**
published in accordance with Art. 153(4) EPC

(43) Date of publication:
**09.07.2025 Bulletin 2025/28**

(21) Application number: **23859848.6**

(22) Date of filing: **07.07.2023**

(51) International Patent Classification (IPC):
**G06N 20/00** (2019.01)

(52) Cooperative Patent Classification (CPC):
**G06N 20/00**

(86) International application number:
**PCT/JP2023/025262**

(87) International publication number:
**WO 2024/048078 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.08.2022 JP 2022138788**

(71) Applicant: **FUJIFILM Corporation
Tokyo 106-8620 (JP)**

(72) Inventors:
• **SATO Masahiro
Ashigarakami-gun, Kanagawa 258-8577 (JP)**
• **UMEKAWA Masao
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54) **INFORMATION PROCESSING METHOD, INFORMATION PROCESSING DEVICE, AND PROGRAM**

(57) Provided are an information processing method, an information processing apparatus, and a program that extract a subset having domain generalization. An information processing method executed by one or more processors, the method including: via the one or more processors, extracting a subset from a dataset to be analyzed under a designated condition; and evaluating domain generalization of the extracted subset. The domain generalization of the subset refers to that, in a classification model or a prediction model trained using the subset, a decrease in classification performance or prediction performance due to a change in a simultaneous probability distribution between an explanatory variable and a response variable due to a change in various conditions of a data generation process is relatively small, or classification performance or prediction accuracy in such a case is high.

FIG. 7

**Description**

**BACKGROUND OF THE INVENTION**

1. Field of the Invention

The present disclosure relates to an information processing method, an information processing apparatus, and a program, and particularly to a technique for constructing a model having domain generalization.

2. Description of the Related Art

A classification model or a prediction model based on data is widely used. For example, disease classification based on a certain medical image is known.

However, in a case where a model trained on data of a certain hospital facility is introduced to another facility, the expected accuracy may not be obtained. This is often due to a domain shift in which the data distributions are different between the learning facility and the introduction facility.

Research on improving robustness against domain shift is called domain generalization, and has been active in recent years. A method of learning a feature amount representation that is invariant regardless of a domain, a meta-learning method of learning for evaluation performance after a pseudo domain shift, and the like are known.

For example, in Vikas Garg, Adam Tauman Kalai, Katrina Ligett, Steven Wu "Learn to Expect the Unexpected: Probably Approximately Correct Domain Generalization" (2021 AISTATS), a method of domain-invariant feature amount selection is proposed. Specifically, for the training data of the plurality of domains, a correlation coefficient between each feature amount (explanatory variable) and a response variable is calculated for each domain, and a feature amount of which an absolute value of the correlation coefficient is equal to or greater than a threshold value in all domains in the training data is selected.

**SUMMARY OF THE INVENTION**

The method of Vikas Garg, Adam Tauman Kalai, Katrina Ligett, Steven Wu "Learn to Expect the Unexpected: Probably Approximately Correct Domain Generalization" (2021 AISTATS) is based on the premise that a classification model or a prediction model with domain generalization can be constructed for the entire data. However, in reality, it is often impossible to do so, and there is a problem that performance is deteriorated.

The present invention has been made in view of such circumstances, and an object of the present invention is to provide an information processing method, an information processing apparatus, and a program for extracting a subset having domain generalization.

In order to achieve the above object, according to a first aspect of the present disclosure, there is provided an information processing method executed by one or more processors, the method comprising: via the one or more processors, extracting a subset from a dataset to be analyzed under a designated condition; and evaluating domain generalization of the extracted subset. The domain generalization of the subset refers to that, in a classification model or a prediction model (classification/prediction model) trained using the subset, a decrease in classification performance or prediction performance due to a change in a simultaneous probability distribution between an explanatory variable and a response variable due to a change in various conditions of a data generation process (domain shift) is relatively small, or classification performance or prediction accuracy in a case of the domain shift is relatively high. According to the present aspect, it is possible to extract a subset having domain generalization, so that it is possible to construct a classification/prediction model having domain generalization.

In the information processing method according to a second aspect of the present disclosure, in the information processing method according to the first aspect, it is preferable that the dataset includes datasets of a plurality of domains, and the evaluation includes, via the one or more processors, extracting a learning subset from a dataset of one or more learning domains among the plurality of domains under the designated condition and training a learning model by using the learning subset, extracting an evaluation subset from a dataset of one or more evaluation domains different from the learning domain among the plurality of domains under the designated condition and evaluating the learning model by using the evaluation subset, and evaluating the domain generalization by using an evaluation result of the learning model.

In the information processing method according to a third aspect of the present disclosure, in the information processing method according to the first aspect or the second aspect, it is preferable that the dataset includes datasets of a plurality of domains, and the evaluation includes, via the one or more processors, extracting a learning subset from a dataset of one or more learning domains among the plurality of domains under the designated condition and training a learning model by using the learning subset, extracting a first evaluation subset different from the learning subset from the dataset of the learning domain under the designated condition, and evaluating the learning model by using the first evaluation subset, extracting a second evaluation subset from a dataset of one or more evaluation domains different from the learning domain among the plurality of domains under the designated condition, and evaluating the learning model by using the second evaluation subset, and evaluating the domain generalization by using a difference between an evaluation result of the first evaluation subset and an evaluation result of the second evaluation subset.

In the information processing method according to a fourth aspect of the present disclosure, in the information processing method according to any one of the first aspect to the third aspect, it is preferable that the dataset includes datasets of a plurality of domains, and the evaluation includes, via the one or more processors, evaluating a degree of association between a feature amount of a subset and a response variable for each of the domains, and assuming that the more the degree of association is relatively high in many domains, the more a domain generality of the feature amount is relatively high, and evaluating that the subset having a larger number of the feature amounts with the relatively high domain generality has a relatively high domain generalization.

In the information processing method according to a fifth aspect of the present disclosure, in the information processing method according to any one of the first aspect to the fourth aspect, it is preferable that the dataset includes datasets of a plurality of domains, and the evaluation includes, via the one or more processors, evaluating a degree of association between a feature amount of a subset and a response variable for each of the domains, and setting a feature amount having a degree of association equal to or greater than a threshold value in a certain number or more of domains as a feature amount having relatively high domain generality, and evaluating the domain generalization of the subset by using the number of the feature amounts having the relatively high domain generality.

In the information processing method according to a sixth aspect of the present disclosure, in the information processing method according to any one of the first aspect to fifth aspect, it is preferable that the dataset includes datasets of a plurality of domains, and the evaluation includes, via the one or more processors, extracting a learning subset from a dataset of one or more learning domains among the plurality of domains under the designated condition and extracting a feature amount set from the learning subset, extracting an evaluation subset from a dataset of one or more evaluation domains different from the learning domain among the plurality of domains under the designated condition, and evaluating the extracted feature amount set by using the evaluation subset, and evaluating the domain generalization of the learning subset by using a proportion of a feature amount that is effective in the evaluation subset among feature amounts of the extracted feature amount set.

In the information processing method of a seventh aspect of the present disclosure, in the information processing method according to the sixth aspect, it is preferable the information processing method further comprises: via the one or more processors, evaluating a degree of association between a feature amount of the learning subset and a response variable for each of the domains; and assuming that the more the degree of association is relatively high in many domains, the more a domain generality of the feature amount is relatively high, and the extracted feature amount set includes the feature amount having the relatively high domain generality.

In the information processing method according to an eighth aspect of the present disclosure, in the information processing method according to the sixth aspect, it is preferable that the information processing method further comprises: via the one or more processors, evaluating a degree of association between a feature amount of the learning subset and a response variable for each of the domains; and setting a feature amount having a degree of association equal to or greater than a certain threshold value in a certain number or more of domains as a feature amount having relatively high domain generality, and the extracted feature amount set includes the feature amount having the relatively high domain generality.

In the information processing method according to a ninth aspect of the present disclosure, in the information processing method according to any one of the first aspect to eighth aspect, it is preferable that the evaluation includes, via the one or more processors, evaluating usefulness of the subset from known usefulness information for each sample in the dataset and a sample included in the subset, and evaluating a subset by combining the usefulness and the domain generalization.

In the information processing method according to a tenth aspect of the present disclosure, in the information processing method according to any one of the first aspect to ninth aspect, it is preferable that the dataset includes datasets of a plurality of domains, and the evaluation includes, via the one or more processors, evaluating domain uniformity indicating a closeness in distribution between a number of data items in the dataset and a number of data items in the subset for each of the domains, and evaluating the subset by combining the domain uniformity and the domain generalization.

In the information processing method according to an eleventh aspect of the present disclosure, in the information processing method according to any one of the first aspect to tenth aspect, it is preferable that the information processing method further comprises: via the one or more processors, training a subset classification model that classifies whether or not data of the dataset is a subset.

In the information processing method according to a twelfth aspect of the present disclosure, in the information processing method according to the eleventh aspect, it is preferable that the evaluation includes, via the one or more processors, evaluating a subset classification performance of the subset classification model, and evaluating the subset by combining the subset classification performance and the domain generalization.

**In** the information processing method according to a thirteenth aspect of the present disclosure, in the information processing method according to any one of the first aspect to the twelfth aspect, it is preferable that the extraction and the evaluation include, via the one or more processors, searching for a subset having a higher domain generalization by repeating an operation of adding or deleting a sample on the subset, from a subset serving as a starting point. The extraction may include repeating an operation of adding or deleting a sample on the subset from a subset serving as a starting point, and the evaluation may include searching for a subset having higher domain generalization.

In the information processing method according to a fourteenth aspect of the present disclosure, in the information processing method according to the thirteenth aspect, it is preferable that the dataset includes datasets of a plurality of domains, and the searching includes, via the one or more processors, searching for the subset by further evaluating any one of usefulness of a subset evaluated from known usefulness information for each sample in the dataset and a sample included in the subset, domain uniformity indicating a closeness in distribution between a number of data items in the dataset and a number of data items in the subset for each of the domains, and a subset classification performance of a subset classification model that classifies whether or not data of the dataset is a subset.

In the information processing method according to a fifteenth aspect of the present disclosure, in the information processing method according to any one of the first aspect to fourteenth aspect, it is preferable that the information processing method further comprises: via the one or more processors, presenting a plurality of different subset conditions; evaluating subsets extracted under each of the plurality of different subset conditions; and extracting a subset under a subset condition having a best evaluation result among the plurality of different subset conditions.

In order to achieve the above object, according to a sixteenth aspect of the present disclosure, there is provided an information processing apparatus comprising: one or more processors; and one or more memories in which a command to be executed by the one or more processors is stored, in which the one or more processors are configured to: extract a subset of a dataset to be analyzed under a designated condition; and evaluate domain generalization of the extracted subset. According to the present aspect, it is possible to extract a subset having domain generalization, so that it is possible to construct a classification/prediction model having domain generalization.

In order to achieve the above object, according to a seventeenth aspect of the present disclosure, there is provided a program causing a computer to implement: a function of extracting a subset of a dataset to be analyzed under a designated condition; and a function of evaluating domain generalization of the extracted subset. According to the present aspect, it is possible to extract a subset having domain generalization, so that it is possible to construct a classification/prediction model having domain generalization.

According to the present disclosure, it is possible to extract a subset having domain generalization.

## BRIEF DESCRIPTION OF THE DRAWINGS

Fig. 1 is an explanatory diagram showing an introduction flow of a classification/prediction system.
Fig. 2 is an explanatory diagram showing a case where a model is trained by domain adaptation.
Fig. 3 is an explanatory diagram showing an example of training data and evaluation data used for machine learning.
Fig. 4 is a graph schematically showing a difference in performance of a model due to a difference in a dataset.
Fig. 5 is a block diagram schematically showing an example of a hardware configuration of an information processing apparatus according to an embodiment.
Fig. 6 is a functional block diagram showing a functional configuration of the information processing apparatus.
Fig. 7 is an explanatory diagram showing classification or prediction according to Embodiment 1.
Fig. 8 is an explanatory diagram showing processing according to Embodiment 2.
Fig. 9 is an explanatory diagram showing processing according to Embodiment 4.
Fig. 10 is an explanatory diagram showing processing according to Embodiment 5.
Fig. 11 is an explanatory diagram showing processing according to Embodiment 6.
Fig. 12 is a graph for explaining the usefulness of the subset.
Fig. 13 is an explanatory diagram showing processing according to Embodiment 7.
Fig. 14 is an explanatory diagram showing processing according to Embodiment 8.
Fig. 15 is an explanatory diagram showing an algorithm of extraction of a subset according to Embodiment 9.
Fig. 16 is a table showing an example of a dataset.
Fig. 17 is a table showing an example of a dataset in which the dataset shown in Fig. 16 is abstracted.
Fig. 18 is a table showing another example of the abstracted dataset.

## DESCRIPTION OF THE PREFERRED EMBODIMENTS

Hereinafter, preferred embodiments of the present invention will be described with reference to the accompanying drawings.

[Construction and Operation of Classification/Prediction Model]

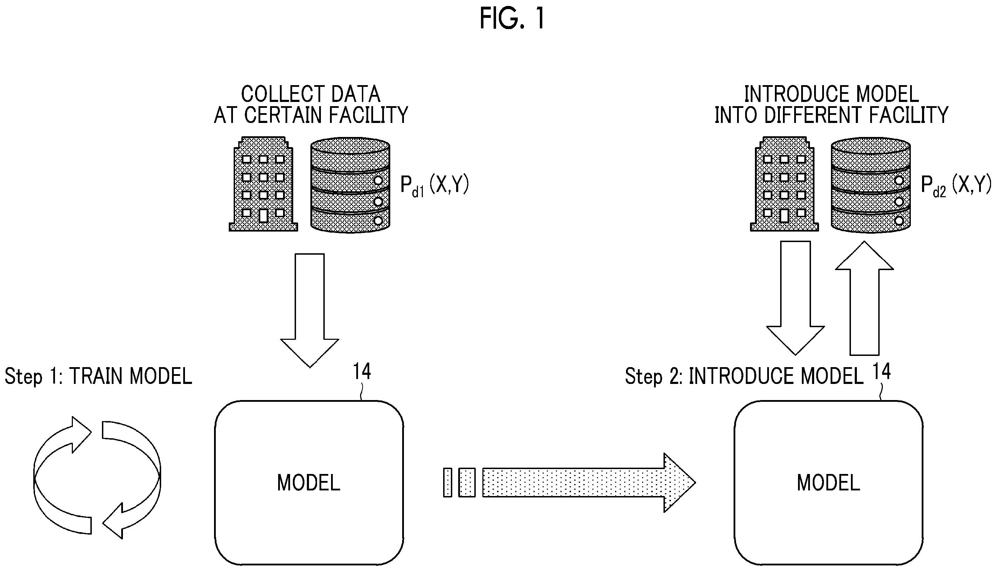

Fig. 1 is an explanatory diagram showing an introduction flow of a classification system or a prediction system (classification/prediction system). Here, a typical flow in a case where a classification/prediction system is introduced into a certain facility is shown. In the introduction of the classification/prediction system, first, a model 14 that performs a target classification task or prediction task (classification/prediction task) is constructed (step 1), and then the constructed model 14 is introduced and operated (step 2). In a case of a machine learning model, "constructing" the model 14 includes training the model 14 using data for training to create a classification/prediction model that satisfies classification performance or prediction performance at a practical level. "Operating" the model 14 is, for example, obtaining an output of the risk of employee retirement from the trained model 14 in response to the input of the dataset related to the employee.

Training data is required for construction of the model 14. In general, the model 14 of the classification/prediction system is trained based on data collected at the introduction destination facility. By performing learning using the data collected from the introduction destination facility, the model 14 learns the behavior of the employee of the introduction destination facility, and can predict the high-accuracy retirement risk for the employee of the introduction destination facility.

However, due to various circumstances, it may not be possible to obtain data on the introduction destination facility. For example, in a case of a document information classification system in an in-house system of a company or an in-hospital system of a hospital, a company that develops a classification model often cannot access the data of the introduction destination facility. In a case where the data of the introduction destination facility cannot be obtained, instead, it is necessary to perform training based on data collected at different facilities.

Fig. 1 shows a classification flow or a prediction flow in a case where data of the introduction destination facility cannot be obtained. In a case where the model 14, which is trained by using the data collected in a facility different from the introduction destination facility, is operated in the introduction destination facility, there is a problem that the prediction accuracy of the model 14 decreases due to differences in user behavior between the facilities.

The problem that the machine learning model does not function well in unknown facilities different from the trained facility is understood as a technical problem, in a broad sense, to improve robustness against a problem of domain shift in which a source domain where the model 14 is trained differs from a target domain where the model 14 is applied. Domain adaptation is problematic related to domain generalization. This is a method of training by using data from both the source domain and the target domain. The purpose of using the data of different domains in spite of the presence of the data of the target domain is to make up for the fact that the amount of data of the target domain is small and insufficient for training.

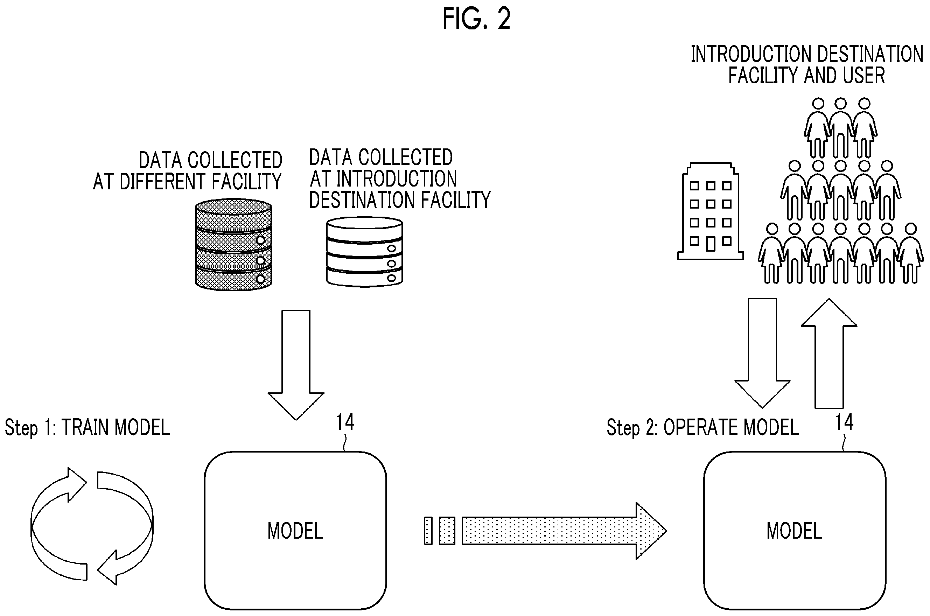

Fig. 2 is an explanatory diagram in a case where the model 14 is trained by domain adaptation. Although the amount of data collected at the introduction destination facility that is the target domain is relatively smaller compared to the data collected at a different facility, the model 14 can also predict with a certain degree of accuracy the behavior of the users in the introduction destination facility by performing a training by using both data.

Domain generalization is a more difficult problem than domain adaptation because the data of the target domain cannot be accessed during training.

[Description of Domain]

In Ivan Cantador, Ignacio Fenandez-Tobias, Shlomo Bwrkovsky, Paolo Cremonesi, Chapter 27: "Cross-domain Recommender System"(2015 Springer), which is a document related to research on domain adaptation in information suggestion, differences in domains are classified into the following four categories.

[1] Item attribute level: For example, a comedy movie and a horror movie are in different domains.
[2] Item type level: For example, a movie and a TV drama series are in different domains.
[3] Item level: For example, a movie and a book are in different domains.
[4] System level: For example, a movie in a movie theater and a movie broadcast on television are in different domains.

The difference between the "facilities" shown in Figs. 1 and 2 corresponds to the [4] system level domain among the above four classifications.

The domain is defined by a simultaneous probability distribution $P(X, Y)$ of the response variable $Y$ and the explanatory variable $X$, and in a case where $Pd1(X, Y) \neq Pd2(X, Y)$, d1 and d2 are different domains. That is, a domain shift occurs.

The simultaneous probability distribution $P(X, Y)$ can be represented by a product of an explanatory variable distribution $P(X)$ and a conditional probability distribution $P(Y|X)$ or a product of a response variable distribution $P(Y)$ and a conditional probability distribution P(Y|X).

$$P(X, Y) = P(Y|X)P(X) = P(X|Y)P(Y)$$

Therefore, in a case where one or more of P(X), P(Y), P(Y|X), and P(X|Y) is changed, the domains are different from each other.

[Reason for Influence of Domain Shift]

Since the classification/prediction model that performs the prediction or classification task performs the inference based on the relationship between the explanatory variable X and the response variable Y, the classification performance or the prediction performance is naturally decreased in a case where P(Y|X) is changed. Further, although minimization of a classification error or a prediction error is performed within training data in a case where machine learning is performed on the classification/prediction model, for example, in a case where the frequency in which the explanatory variable becomes $X = X\_1$ is greater than the frequency in which the explanatory variable becomes $X = X\_2$, that is, in a case where $P(X = X\_1) > P(X = X\_2)$, the data of $X = X\_1$ is greater than the data of $X = X\_2$, thereby error decrease for $X = X\_1$ is trained in preference to error decrease for $X = X\_2$. Therefore, even in a case where P(X) changes between facilities, the classification performance or the prediction performance is degraded.

[Example of Domain Shift]

The domain shift can be a problem for models of various tasks. For example, regarding a model that predicts the retirement risk of an employee, a domain shift may be a problem in a case where a prediction model, which is trained by using data of a certain company, is operated by another company.

Further, in a model that predicts an antibody production amount of a cell, a domain shift may be a problem in a case where a model, which is trained by using data of a certain antibody, is operated for another antibody. Further, for a model that classifies the voice of customer (VOC), for example, a model that classifies VOC into "product function", "support response", and "other", a domain shift may be a problem in a case where a classification model, which is trained by using data related to a certain product, is operated for another product.

[Typical Pattern of Domain Shift]

[Covariate shift] A case where explanatory variable distributions P(X) are different is called a covariate shift. For example, a case where distributions of user attributes are different between datasets, more specifically, a case where a gender ratio is different, and the like correspond to the covariate shift.

[Prior probability shift] A case where response variable distributions P(Y) are different is called a prior probability shift. For example, a case where an average browsing rate and an average purchase ratio differ between datasets corresponds to the prior probability shift.

[Concept shift] A case where conditional probability distributions P(Y|X) and P(X|Y) are different is called a concept shift. For example, in a case where a probability that a research and development department of a certain company reads data analysis materials is assumed as P(Y|X), and the probability differs between datasets, this case corresponds to the concept shift.

Research on domain adaptation or domain generalization includes assuming one of the above-mentioned patterns as a main factor and looking at dealing with P(X, Y) changing without specifically considering which pattern is a main factor. In the former case, there are many cases in which a covariate shift is assumed.

[Subset Extraction]

In a case where it is impossible to construct a classification/prediction model having domain generalization for the entire data, by having a subset in the data appropriately extracted, a classification/prediction model having domain generalization can be constructed in the subset. The present disclosure proposes an appropriate subset extraction method.

The subset is a subset of the dataset to be analyzed. As an example of the subset extraction condition, for example, for the training of a model that predicts the risk of an employee retirement, a subset in which only the data of the employees in the "sales department" of the data of a certain company is extracted can be used. In a case where a prediction model trained using a subset of "sales department" is operated in another company, the subset is domain-agnostic in the sales department.

In addition, for the model that predicts the antibody production amount of the cell, a subset in which only the data of the antibody of "cell size is equal to or greater than a certain value" is extracted from the data of a certain antibody can be used. In a case where a model trained using a subset of "cell size is equal to or greater than a certain value" is operated with another antibody, the subset is domain-agnostic as long as the "cell size is equal to or greater than a certain value".

In addition, for a model that classifies the voice of the customer into "product function", "support response", and "other", a subset in which only the data of the customer of the "cat owner" among the data related to a certain product is extracted can be used. In a case where a classification model trained using a subset of "cat owner" is operated with another product, the subset is domain-agnostic as long as the subset is "cat owner".

[Regarding Generalization]

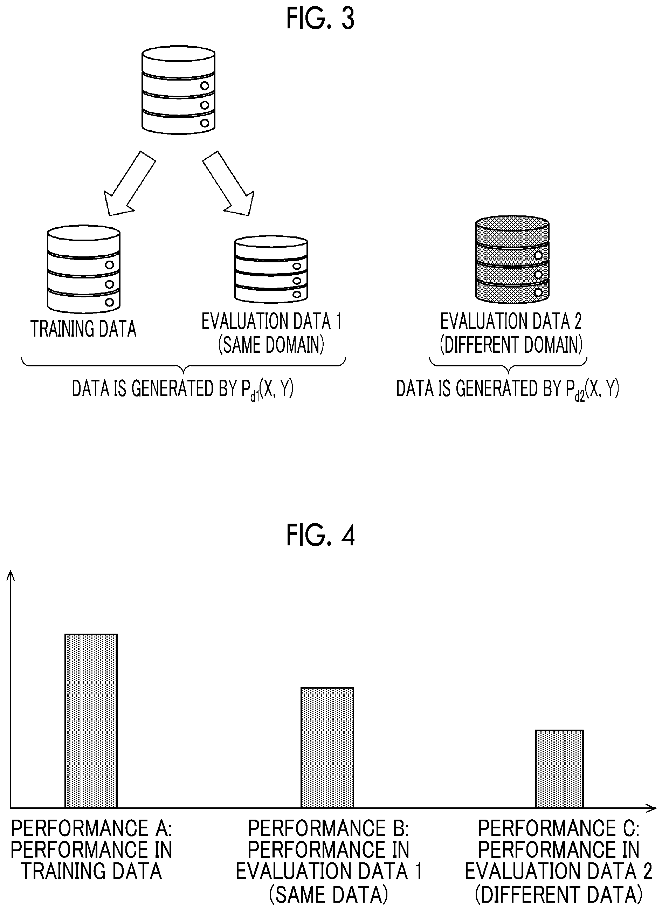

Fig. 3 is an explanatory diagram showing an example of the training data and the evaluation data used for the machine learning. The dataset obtained from the simultaneous probability distribution Pd1(X, Y) of a certain domain d1 is divided into training data and evaluation data. The evaluation data of the same domain as the training data is referred to as "first evaluation data" and is referred to as "evaluation data 1" in Fig. 3. Further, a dataset, which is obtained from a simultaneous probability distribution Pd2(X, Y) of a domain d2 different from the domain d1, is prepared and is used as the evaluation data. The evaluation data of the domain different from the training data is referred to as "second evaluation data" and is referred to as "evaluation data 2" in Fig. 3.

The model 14 is trained by using the training data of the domain d1, and the performance of the model 14, which is trained by using each of the first evaluation data of the domain d1 and the second evaluation data of the domain d2, is evaluated.

Fig. 4 is a graph schematically showing a difference in performance of the model due to a difference in the dataset. Assuming that the performance of the model 14 in the training data is defined as performance A, the performance of the model 14 in the first evaluation data is defined as performance B, and the performance of the model 14 in the second evaluation data is defined as performance C, normally, a relationship is represented such that performance A> performance B> performance C, as shown in Fig. 4.

High generalization performance of the model 14 generally indicates that the performance B is high, or indicates that a difference between the performances A and B is small. That is, the aim is to achieve high prediction performance even for unlearned data without over-fitting to the training data.

In the context of domain generalization in the present specification, it means that the performance C is high or a difference between the performance B and the performance C is small. In other words, the aim is to achieve high performance consistently even in a domain different from the domain used for the training.

In addition, in the present specification, the domain generalization of the training data indicates that, in a classification/prediction model trained using the training data, a decrease in classification performance or prediction performance due to a change in the simultaneous probability distribution between the explanatory variable and the response variable due to a change in various conditions of the data generation process (domain shift) is relatively small, or the classification performance or prediction accuracy in a case of the domain shift is relatively high. That is, in a case where the domain generalization of the model 14 is relatively high, the training data used for training the model 14 has relatively high domain generalization.

Strictly speaking, the domain generalization of the training data is the presence of a classification/prediction model with relatively little performance degradation, in other words, the performance of the classification/prediction model with the smallest performance degradation with respect to the domain shift among the classification/prediction model candidates is the domain generalization. For example, in a case where it is determined to be cancer in a case where one gene is selected among the gene A, the gene B, and the gene C, and the expression level is equal to or higher than the average value of all the training data, the classification/prediction model is uniquely determined by selecting the gene (= feature amount). Therefore, there are three model candidates: a model A using the gene A, a model B using the gene B, and a model C using the gene C.

Here, it is assumed that in a case where there is no domain shift, the classification accuracy of all of the model A, the model B, and the model C is 90%, and in a case where there is a domain shift, the classification accuracy of the model A, the model B, and the model C is 70%, 80%, and 50%, respectively. In this case, the performance degradation of the model A, the model B, and the model C due to the domain shift is -20%, -10%, and -40%, respectively, and -10% of the model B, which is the best model, is the domain generalization.

However, in a case where the classification accuracies of the model A, the model B, and the model C in a case where there is no domain shift are 75%, 90%, and 80%, respectively, the performance deterioration due to the domain shift is -5%, -10%, and -30%, respectively. In this case, -5% of the model A having the minimum performance degradation may be defined as the domain generalization performance, or 80% of the model B having the maximum accuracy in a case of the domain shift may be defined as the domain generalization performance.

It is considered that, in a case of a universal characteristic, the universal characteristic can be generally applied.

Therefore, there is a relationship that the subset leads to domain generalization in a case where the subset is domain-agnostic. For example, in a case where a characteristic that an expression of a certain gene leads to cancer is universal regardless of a race (domain), a unit for determining cancer by the expression of the gene can be used universally regardless of the race.

[Outline of Information Processing Apparatus]

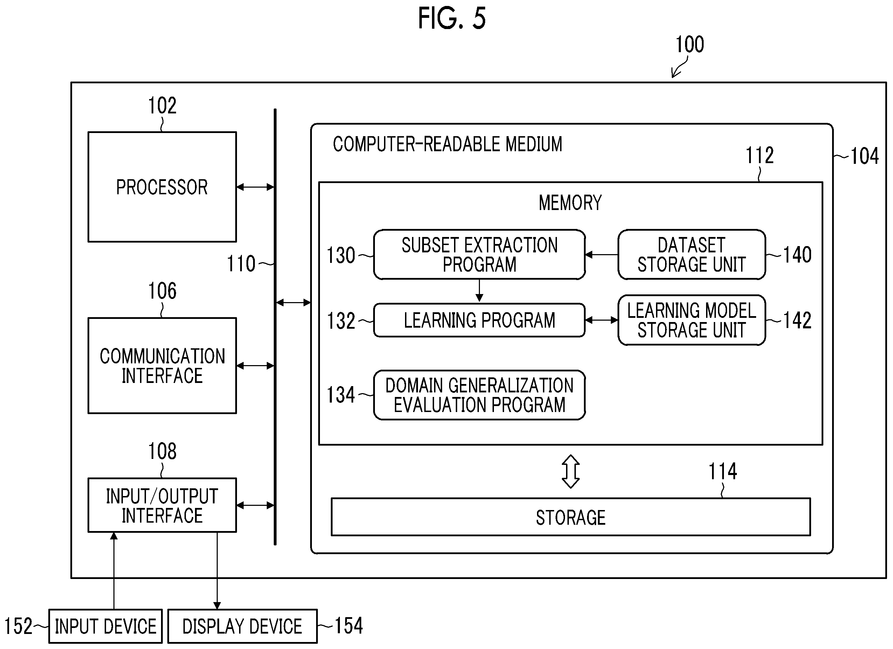

Fig. 5 is a block diagram schematically showing an example of a hardware configuration of an information processing apparatus 100 according to an embodiment. The information processing apparatus 100 comprises a function of extracting a subset of a dataset to be analyzed under a designated condition, and a function of evaluating domain generalization.

The information processing apparatus 100 can be implemented by using hardware and software of a computer. The physical form of the information processing apparatus 100 is not particularly limited, and may be a server computer, a workstation, a personal computer, a tablet terminal, or the like. Although an example of realizing a processing function of the information processing apparatus 100 using one computer will be described here, the processing function of the information processing apparatus 100 may be implemented by a computer system configured by using a plurality of computers.

The information processing apparatus 100 includes a processor 102, a computer-readable medium 104 that is a non-transitory tangible object, a communication interface 106, an input/output interface 108, and a bus 110.

The processor 102 includes a central processing unit (CPU). The processor 102 may include a graphics processing unit (GPU). The processor 102 is connected to the computer-readable medium 104, the communication interface 106, and the input/output interface 108 via the bus 110. The processor 102 reads out various programs, data, and the like stored in the computer-readable medium 104 and executes various processes. The term program includes the concept of a program module and includes commands conforming to the program.

The computer-readable medium 104 is, for example, a storage device including a memory 112 which is a main memory and a storage 114 which is an auxiliary storage device. The storage 114 is configured using, for example, a hard disk drive (HDD) device, a solid state drive (SSD) device, an optical disk, a photomagnetic disk, a semiconductor memory, or an appropriate combination thereof. Various programs, data, and the like are stored in the storage 114.

The memory 112 is used as a work area of the processor 102 and is used as a storage unit that temporarily stores the program and various types of data read from the storage 114. By loading the program that is stored in the storage 114 into the memory 112 and executing commands of the program by the processor 102, the processor 102 functions as a unit for performing various processes defined by the program.

The memory 112 stores an extraction program 130, a learning program 132, an evaluation program 134, various programs, various types of data, and the like executed by the processor 102.

The extraction program 130 is a program that acquires a dataset and executes processing of extracting a subset, which is a subset of the dataset to be analyzed, from the acquired dataset.

The learning program 132 is a program that causes the processor 102 to execute processing of learning a classification/prediction model that performs classification or prediction for each subset extracted by the extraction program 130.

The evaluation program 134 is a program that causes the computer to execute processing of evaluating the domain generalization of the subset extracted by the extraction program 130. The evaluation program 134 may execute processing of evaluating the domain generalization of the classification/prediction model trained by the learning program 132.

The memory 112 includes a dataset storage unit 140 and a learning model storage unit 142. The dataset storage unit 140 is a storage area in which the dataset collected at the learning facility is stored. The learning model storage unit 142 is a storage area in which the classification/prediction model trained by the learning program 132 is stored.

The communication interface 106 performs communication processing with an external device by wire or wirelessly and exchanges information with the external device. The information processing apparatus 100 is connected to a communication line (not shown) via the communication interface 106. The communication line may be a local area network, a wide area network, or a combination thereof. The communication interface 106 can play a role of a data acquisition unit that receives input of various data such as the original dataset.

The information processing apparatus 100 may include an input device 152 and a display device 154. The input device 152 and the display device 154 are connected to the bus 110 via the input/output interface 108. The input device 152 may be, for example, a keyboard, a mouse, a multi-touch panel, or other pointing device, a voice input device, or an appropriate combination thereof. The display device 154 may be, for example, a liquid crystal display, an organic electro-luminescence (OEL) display, a projector, or an appropriate combination thereof. The input device 152 and the display device 154 may be integrally configured as in the touch panel, or the information processing apparatus 100, the input device 152, and the display device 154 may be integrally configured as in the touch panel type tablet terminal.

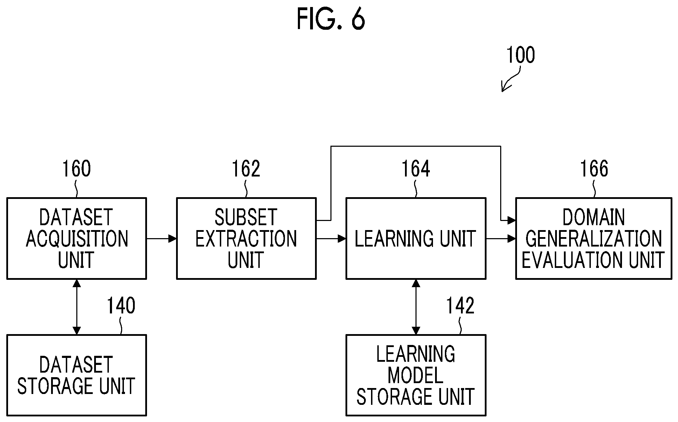

Fig. 6 is a functional block diagram showing a functional configuration of an information processing apparatus 100. The information processing apparatus 100 includes a dataset acquisition unit 160, a subset extraction unit 162, a learning unit 164, and a domain generalization evaluation unit 166.

The dataset acquisition unit 160 acquires the dataset collected at the learning facility from the dataset storage unit 140. The dataset is a set of data for each domain, and includes an explanatory variable and a response variable.

The subset extraction unit 162 extracts a subset under a designated condition from the dataset acquired by the dataset acquisition unit 160. The subset includes all the feature amounts (explanatory variables) and the response variables of the dataset. That is, the subset extraction unit 162 does not narrow down the feature amount. The condition for extraction may be designated by the user.

The learning unit 164 generates a classification/prediction model that performs classification or prediction for each subset.

The domain generalization evaluation unit 166 is a program that causes the processor 102 to execute processing of evaluating the domain generalization of the subset extracted by the subset extraction unit 162. The domain generalization evaluation unit 166 may evaluate the domain generalization of the classification/prediction model trained by the learning unit 164.

[Embodiment 1]

The information processing apparatus 100 performs an information processing method of extracting a subset from a dataset to be analyzed under a designated condition and evaluating domain generalization of the extracted subset.

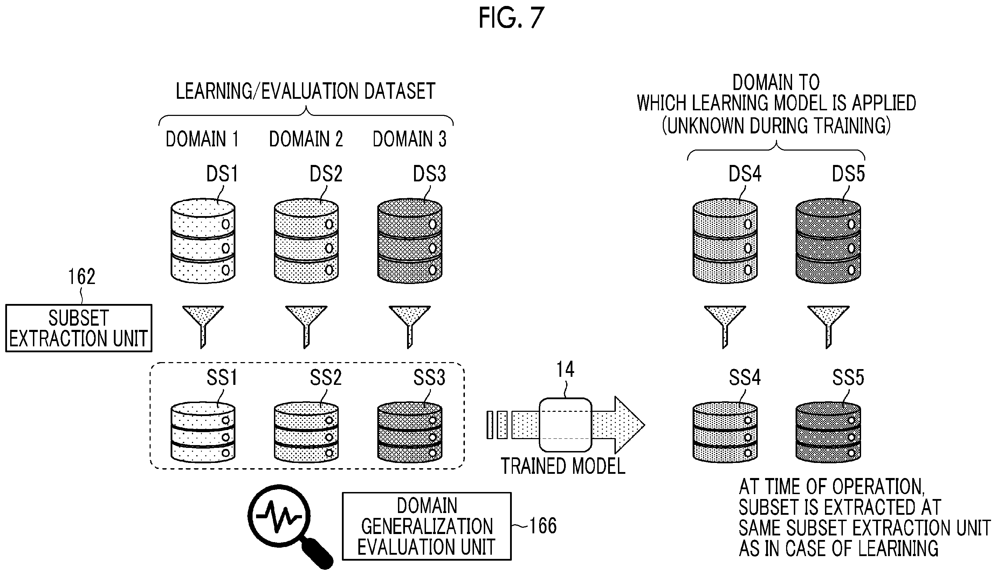

Fig. 7 is an explanatory diagram showing classification or prediction according to Embodiment 1. A learning or evaluation dataset (learning/evaluation dataset) which is a dataset to be analyzed in Embodiment 1 includes a dataset DS1 of a domain 1, a dataset DS2 of a domain 2, and a dataset DS3 of a domain 3. The domain 1, the domain 2, and the domain 3 are different domains from each other.

The subset extraction unit 162 extracts a subset from the dataset under a designated condition. That is, the subset extraction unit 162 extracts the subset SS1 from the dataset DS1, the subset SS2 from the dataset DS2, and the subset SS3 from the dataset DS3.

The domain generalization evaluation unit 166 evaluates the domain generalization of the subsets SS1, SS2, and SS3.

In a case where the subsets SS1, SS2, and SS3 have domain generalization, the learning unit 164 generates the model 14 that performs classification or prediction using the subsets SS1, SS2, and SS3. Since the subsets SS1, SS2, and SS3 have domain generalization, a model 14 having domain generalization can be constructed.

In addition, in the present embodiment, there are datasets DS4 and DS5 of domains to which the learning model is applied. This domain is an unknown domain during the training of the model 14.

The subset extraction unit 162 extracts a subset from the dataset under a designated condition. That is, the subset extraction unit 162 extracts the subset SS4 from the dataset DS4 and extracts the subset SS5 from the dataset DS5. The designated condition here is the same condition as the condition for extracting the subsets SS1 to SS3 from the datasets DS1 to DS3.

By applying the model 14 to the subsets SS4 and SS5, it is possible to perform classification or prediction with high accuracy.

[Embodiment 2]

The information processing apparatus 100 may divide a dataset of a plurality of domains for each domain, extract a learning subset from a dataset of one or more learning domains among the plurality of domains under a designated condition, train a learning model by using the learning subset, extract an evaluation subset from a dataset of one or more evaluation domains different from the learning domains among the plurality of domains under a designated condition, evaluate the learning model by using the evaluation subset, and evaluate the domain generalization by using an evaluation result of the learning model.

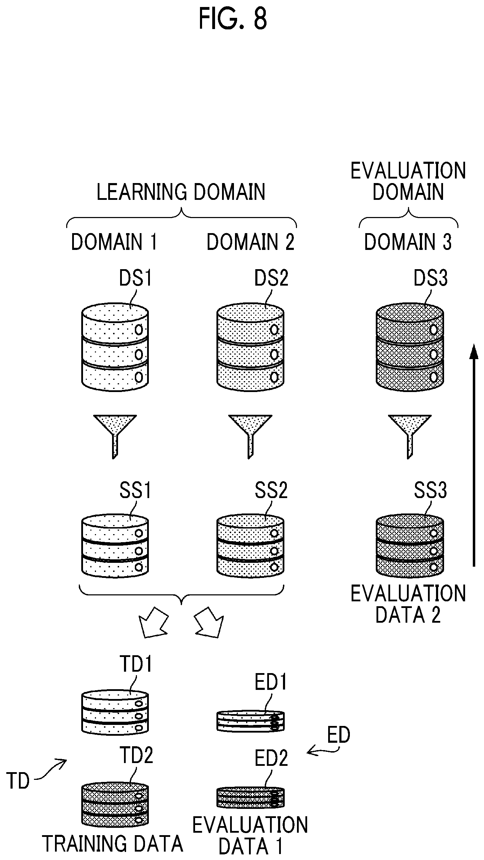

Fig. 8 is an explanatory diagram showing processing according to Embodiment 2. Here, the domain 1 and the domain 2 are the learning domains, and the domain 3 is the evaluation domain.

The subset extraction unit 162 extracts the subset SS1 from the dataset DS1 of the domain 1, and the subset SS2 from the dataset DS2 which are the learning domains, under the designated condition.

In addition, the subset extraction unit 162 divides the subsets SS1 and SS2 into the training data TD and the first evaluation data ED, respectively. That is, the subset extraction unit 162 divides the subset SS1 into the training data TD1 and the evaluation data ED1, and divides the subset SS2 into the training data TD2 and the evaluation data ED2. The training data TD includes training data TD1 and training data TD2, and the first evaluation data ED includes evaluation data ED1 and evaluation data ED2.

Further, the subset extraction unit 162 extracts the subset SS3 from the dataset DS3 of the domain 3, which is the evaluation domain, under the designated condition. The subset SS3 corresponds to the second evaluation data.

The learning unit 164 performs learning of the model 14 using training data TD (an example of the "learning subset").

The domain generalization evaluation unit 166 evaluates the domain generalization by the performance C shown in Fig. 4. That is, the domain generalization evaluation unit 166 evaluates the domain generalization of the model 14 that has been trained by using the subset SS3 (an example of the "evaluation subset"), which is the second evaluation data.

The information processing apparatus 100 may divide the dataset of the plurality of domains for each domain, extract a learning subset from the dataset of one or more learning domains among the plurality of domains under a designated condition, train a learning model by using the learning subset, extract a first evaluation subset different from the learning subset under a designated condition from the dataset of the learning domain, evaluate the learning model by using the first evaluation subset, extract a second evaluation subset under a designated condition from the dataset of one or more evaluation domains different from the learning domain among the plurality of domains, evaluate the learning model by using the second evaluation subset, and evaluate the domain generalization by using a difference between an evaluation result of the first evaluation subset and an evaluation result of the second evaluation subset.

That is, the domain generalization evaluation unit 166 evaluates the domain generalization based on the difference between the performance B and the performance C shown in Fig. 4. In this case, the domain generalization evaluation unit 166 may evaluate the domain generalization by using a difference between the performance of the trained model 14 using the first evaluation data ED (an example of the "first evaluation subset") and the performance of the trained model 14 using the subset SS3 (an example of the "second evaluation subset") which is the second evaluation data. Here, the smaller the difference, the higher the domain generalization.

The domain generalization evaluation unit 166 may evaluate the domain generalization by comprehensively determining both the performance C shown in Fig. 4 and the difference between the performance B and the performance C shown in Fig. 4.

[Embodiment 3]

The information processing apparatus 100 may divide the dataset of the plurality of domains for each domain, evaluate the degree of association between the feature amount (explanatory variable) and the response variable of the subset of the dataset divided for each domain, set the feature amount having the degree of association equal to or greater than a certain threshold value in a certain number or more of domains as the feature amount having relatively high domain generality, and evaluate the domain generalization of the subset by using the number of the feature amounts having relatively high domain generality.

In the present embodiment, S is a set of samples of data and is represented as

$$S = \{1, 2, ..., i, ..., |S|\}.$$

F is a set of feature amounts and is represented by

$$F = \{1, 2, ..., k, ..., |F|\}.$$

D is a set of domains and is represented by

$$D = \{1, 2, ..., d, ..., |D|\}.$$

Each sample i includes a feature vector x' ($x' \in R^{|F|}$) included in a real vector of |F| dimensions and a response variable yi. The value of the k-th feature amount of the sample i is represented by

$$x_k^i.$$

Each sample is included in any one domain.

A domain of the sample i is represented by di, and di $\in$ D. The subset G is extracted from the dataset S, and

$$v_k^d(G)$$

, in which $G \subset S$, indicates a correlation between the k-th feature amount of the domain d and the response variable. For example, in $G \cap [i| d_i = d]$, the Pearson correlation coefficient is obtained between

$$x_k^i$$

, which is the value of the k-th feature amount of the response variable yi and the sample i.

A subset $F^{DG} \subset G$ of feature amounts having domain generality is defined as in the following Expression (1).

$$F^{DG}(G) = \left\{ k \,\middle|\, \max\left( \sum_d \mathbb{1}\left(v_k^d(G) \geq \theta\right), \sum_d \mathbb{1}\left(v_k^d(G) \leq -\theta\right) \right) \geq m \right\}, \quad \cdots \text{Expression (1)}$$

Here,

$\mathbb{1}$ is an indicator function that is 1 in a case where a correlation between the feature amount and the response variable is equal to or greater than $\theta$ or equal to or less than $-\theta$, and is 0 in other cases. $\theta$ is a certain threshold value, and is, for example, $\theta = 0.8$. m is a certain number, for example, the total number of domains is 5, and m = 4. The domain generalization of the subset G is evaluated by the number of feature amounts of $F^{DG}(G)$, that is, $|F^{DG}(G)|$.

The information processing apparatus 100 may divide the dataset of the plurality of domains for each domain, evaluate the degree of association between the feature amount of the subset of the dataset and the response variable divided for each domain, set such that the more the degree of association is relatively high in many domains, the more the domain generality of the feature amount is relatively high, and evaluate that the subset having a larger number of the feature amounts with the relatively high domain generality has a relatively high domain generalization.

That is, more generally, $|F^{DG}(G)|$ is preferably larger as $\theta$ and m are larger. Such an evaluation method can be represented by the following Expression (2).

$$\sum_{m=2}^{|D|} m^\alpha \int_0^\Theta \theta^\beta \left| F^{DG}(G) \right| d\theta \quad \cdots \text{Expression (2)}$$

Here, $\alpha$ and $\beta$ are scaling factors for weighting as the values of m and $\theta$ are larger.

The feature amount selection method is various, but is classified into a filter method, a wrapper method, and an embedded method as described in G Chandrashekar, F Sahin, Computers & Electrical Engineering, "A survey on feature selection methods" (2014 Elsevier), and Y Saeys, I Inza, P Larranaga "A review of feature selection techniques in bioinformatics" (bioinformatics, 2007).

The filter method evaluates the relevance between the feature amount and the response variable independently of the classification model. In the wrapper method, the feature amount is evaluated by the performance of a specific classification model. In the embedded method, the feature amount selection is intrinsically incorporated into the algorithm of the classification model. For example, a decision tree, Lasso, or the like is used.

The filter method is classified into univariate methods that evaluate the feature amounts one by one and multivariate methods that evaluate the feature amounts with a feature amount set. The method using Pearson correlation is one of univariate methods of the filter method. As a matter of course, another univariate method may be used. It is also easy to extend to a multivariate method and a wrapper method. In that case, the degree of association v is defined for the feature amount set, and the feature amount set F' satisfying the condition of Expression (3) is the feature amount set having domain generality.

$$\left( \sum_d \mathbb{1}\left( v_{F'}^d(G) \geq \theta \right) \right) \geq m \quad \cdots \text{Expression (3)}$$

[Embodiment 4]

The information processing apparatus 100 may divide the dataset of the plurality of domains for each domain, extract a learning subset from the dataset of one or more learning domains among the plurality of domains under a designated condition, extract a feature amount set from the learning subset, extract an evaluation subset from the dataset of one or more evaluation domains different from the learning domain among the plurality of domains under a designated condition, evaluate the extracted feature amount set by using the evaluation subset, and evaluate the domain generalization of the learning subset by using a proportion of a feature amount that is effective in the evaluation subset among the feature amounts of the extracted feature amount set.

The information processing apparatus 100 may evaluate the degree of association between the feature amount of the learning subset and the response variable for each domain, set such that the more the degree of association is relatively high in many domains, the more the domain generality of the feature amount is relatively high, and evaluate that the extracted feature amount set may include the feature amount with the relatively high domain generality.

The information processing apparatus 100 may evaluate the degree of association between the feature amount of the learning subset and the response variable for each domain, and may set the feature amount having the degree of association equal to or greater than a certain threshold value in a certain number or more of domains as the feature amount having relatively high domain generality, and the extracted feature amount set may include the feature amount having relatively high domain generality.

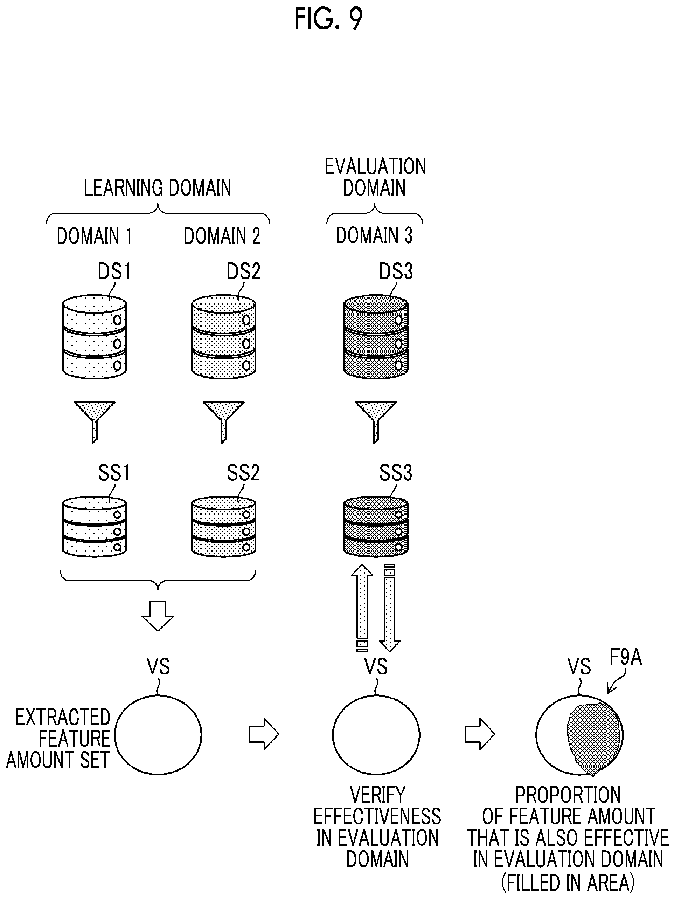

Fig. 9 is an explanatory diagram showing processing according to Embodiment 4. Here, the domain 1 and the domain 2 are the learning domains, and the domain 3 is the evaluation domain.

The subset extraction unit 162 extracts the subset SS1 from the dataset DS1 of the domain 1, and the subset SS2 from the dataset DS2 which are the learning domains, under the designated condition. In addition, the subset extraction unit 162 extracts a subset SS3 from the dataset DS3 of the domain 3, which is the evaluation domain, under the designated condition.

The domain generalization evaluation unit 166 extracts a feature amount set VS from all the feature amounts included in the subset SS1 and the subset SS2 (an example of a "learning subset"). The feature amount set VS is a subset of the explanatory variables of the subset SS1 and the subset SS2, or a feature amount obtained from the subset.

The domain generalization evaluation unit 166 basically extracts the feature amount set VS based on a criterion based on the degree of association between the response variable and the explanatory variable. For example, in a case where the height is the response variable and the expression level of each of 10,000 genes is the explanatory variable, a Pearson correlation coefficient between each gene expression level and the height is calculated, and a gene having a correlation coefficient equal to or greater than a certain value is extracted. **In a** case where the explanatory variable is a binary variable such as whether or not the cancer is present, rather than a continuous value such as height, the selection is performed based on a p value of the t test, an area under the curve (AUC), or the like. These correspond to the above-described filter method.

**In** the case of the wrapper method, the performance of the classification model is evaluated, and for example, the classification accuracy in a case where each gene is added to the explanatory variable and a case where each gene is not added to the explanatory variable are compared by using logistic regression model, and those having a large difference (those having a large improvement in accuracy by being added to the explanatory variable) are selected.

However, in a case where the feature amount set VS is extracted in consideration of the domain generality in the learning domain, for example, the feature amount set VS having the Pearson correlation coefficient of equal to or greater than a certain value is extracted in both the domain 1 and the domain 2.

**In** addition, the domain generalization evaluation unit 166 verifies the effectiveness of the feature amount set VS by using the subset SS3 (an example of an "evaluation subset").

The following methods can be considered as the method of verifying the effectiveness.

· The same feature amount extraction as the learning domain is performed in the evaluation domain, and the overlap is taken.
· The positive-negative of the relevance between the learning domain and the feature amount is the same. That is, in a case where there is a positive correlation in the learning domain, there is at least a positive correlation in the evaluation domain.

F9A shown in Fig. 9 shows the feature amount set VS in a case where the proportion of the feature amount that is also effective in the evaluation domain is filled in. For example, with respect to a case where the number of extracted feature amounts is 20 and the number of effective feature amounts is 10, the proportion of the effective feature amounts is higher in a case where the number of extracted feature amounts is 15 and the number of effective feature amounts is 9. Therefore, the latter subset has relatively high domain generalization.

[Embodiment 5]

The information processing apparatus 100 may present a plurality of different subset conditions, evaluate subsets extracted under each of the plurality of different subset conditions, and extract a subset under a subset condition having the best evaluation result among the plurality of different subset conditions.

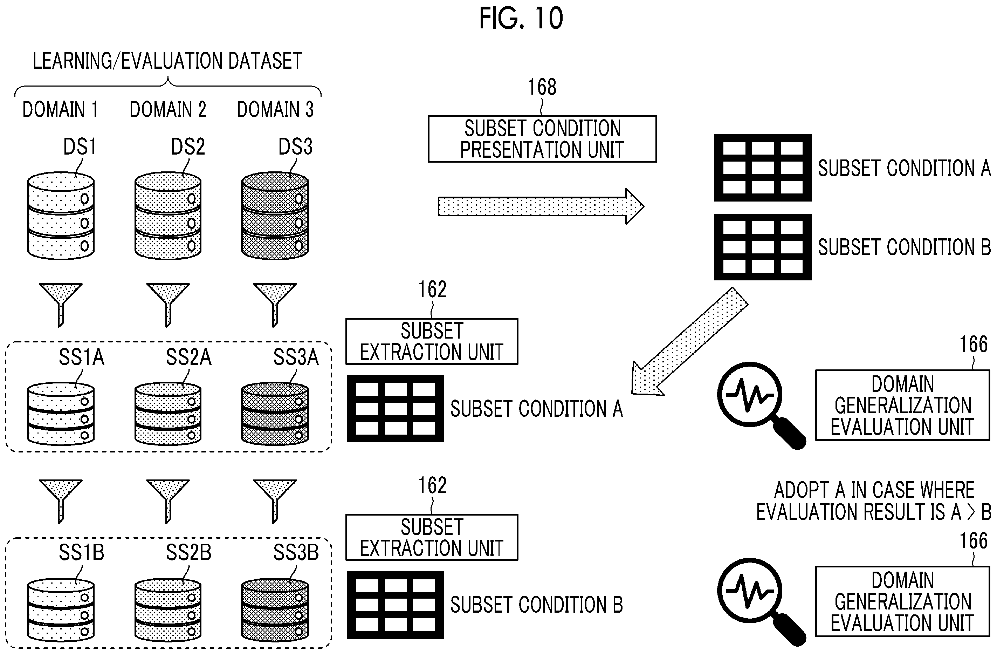

Fig. 10 is an explanatory diagram showing processing according to Embodiment 5. The information processing apparatus 100 comprises a subset condition presentation unit 168. The subset condition presentation unit 168 presents a plurality of different subset conditions (an example of "designation"). Here, the dataset DS1 of the domain 1, the dataset DS2 of the domain 2, and the dataset DS3 of the domain 3 are provided as the learning/evaluation dataset.

In the example shown in Fig. 10, the subset condition presentation unit 168 presents subset condition A and subset condition B which are different from each other. In a subset used for a model that predicts the antibody production amount of cells, for example, the subset condition A is "cell size is 10 $\mu$m or more" and the subset condition B is "cell size is 15 $\mu$m or more". That is, the subset condition A and subset condition B, which are different from each other, are defined by different values (attributes) of "10 $\mu$m or more" and "15 $\mu$m or more" for the same feature amount item of "cell size".

The subset extraction unit 162 extracts subsets SS1A, SS2A, and SS3A from the datasets DS1, DS2, and DS3, respectively, under the subset condition A. In addition, the subset extraction unit 162 extracts subsets SS1B, SS2B, and SS3B from the datasets DS1, DS2, and DS3, respectively, under the subset condition B.

The domain generalization evaluation unit 166 adopts a subset condition with a good evaluation result among the subset condition A and the subset condition B. The subset extraction unit 162 extracts a subset under the adopted subset condition.

Here, the domain 1 and the domain 2 are set as a learning domain, and the domain 3 is set as an evaluation domain. The learning unit 164 generates the model 14 using the subsets SS1A and SS2A of the subsets under the subset condition A as the training data, and generates the model 14 using the subsets SS1B and SS2B of the subsets under the subset condition B as the training data.

In addition, the domain generalization evaluation unit 166 evaluates the model 14 of the subset condition A by using a subset SS3A of the subsets under the subset condition A as evaluation data, and evaluates the model 14 of the subset condition B by using a subset SS3B of the subsets under the subset condition B as evaluation data. As a result, the subset conditions A and B can be evaluated.

The subset conditions A and B may be evaluated using the feature amount set as in Embodiment 4. In this case, the domain generalization evaluation unit 166 extracts the feature amount set from the subset SS 1A and the subset SS2A, and verifies the effectiveness of the feature amount set by using the subset SS3A. In addition, the domain generalization evaluation unit 166 extracts the feature amount set from the subset SS1B and the subset SS2B, and verifies the effectiveness of the feature amount set by using the subset SS3B.

[Embodiment 6]

The information processing apparatus 100 may evaluate the usefulness of the subset from the known usefulness information for each sample in the dataset and the samples included in the subset, and may evaluate the subset by combining the usefulness and the domain generalization.

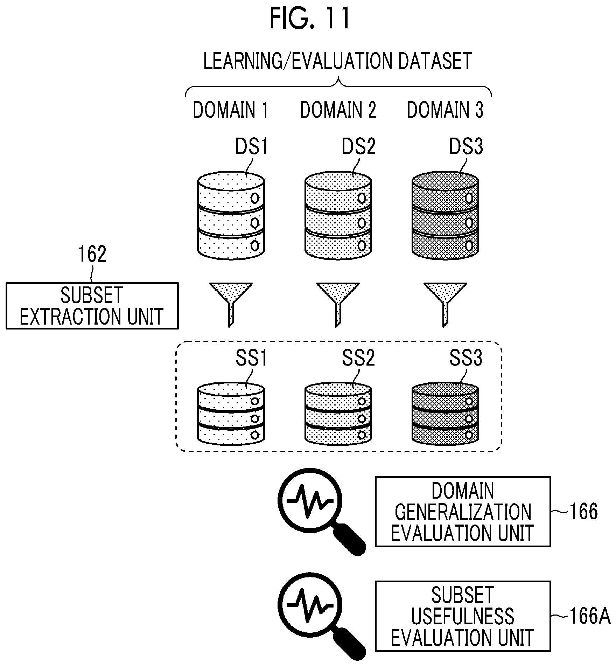

Fig. 11 is an explanatory diagram showing processing according to Embodiment 6. The domain generalization evaluation unit 166 comprises a subset usefulness evaluation unit 166A. The subset usefulness evaluation unit 166A evaluates the usefulness of the subset from the known usefulness information for each sample in the dataset and the samples included in the subset.

As shown in Fig. 11, here, a dataset DS1 of a domain 1, a dataset DS2 of a domain 2, and a dataset DS3 of a domain 3 are provided as a learning/evaluation dataset. The subset extraction unit 162 extracts subsets SS1, SS2, and SS3 from the datasets DS1, DS2, and DS3, respectively.

The domain generalization evaluation unit 166 evaluates the domain generalization of the subsets SS1, SS2, and SS3.

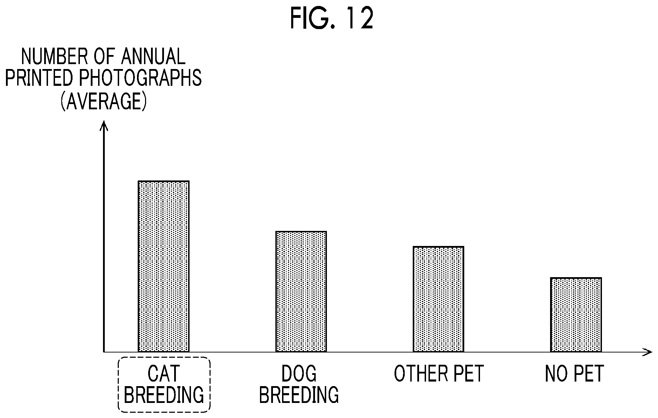

In addition, the subset usefulness evaluation unit 166A evaluates the usefulness of the subsets SS1, SS2, and SS3. Fig. 12 is a graph for explaining the usefulness of the subset. Here, in the VOC classification of the camera, it is assumed that the user with a larger number of annual photographic print is better, that is, the sample with relatively high usefulness. The subset usefulness evaluation unit 166A calculates the average of the number of annual photographic print of each user of "cat breeding", "dog breeding", "other pet", and "no pet" for the subsets SS1, SS2, and SS3, and relatively evaluates the usefulness of the subset higher as the average is larger. In the example shown in Fig. 12, the usefulness is high in the order of "cat breeding", "dog breeding", "other pets", and "no pet".

In addition, in a case of the VOC classification, the subset usefulness evaluation unit 166A may evaluate that the more the number of royal customers, the relatively higher the usefulness of the subset. In the case of antibody production prediction, the subset usefulness evaluation unit 166A may evaluate that the higher the cell proliferation rate, the relatively higher the usefulness of the subset.

The subset usefulness evaluation unit 166A takes the average of the usefulness of the individual samples for the samples belonging to the subsets SS1, SS2, and SS3, and uses the average as the usefulness of the subsets SS1, SS2, and SS3.

The domain generalization evaluation unit 166 evaluates the subsets SS1, SS2, and SS3 by combining the usefulness and the domain generalization evaluated in the subset usefulness evaluation unit 166A.

[Embodiment 7]

The dataset may include datasets of a plurality of domains, and the information processing apparatus 100 may evaluate domain uniformity indicating a closeness in distribution between the number of data items in the dataset for each domain and the number of data items in the subset, and may evaluate the subset by combining the domain uniformity and the domain generalization.

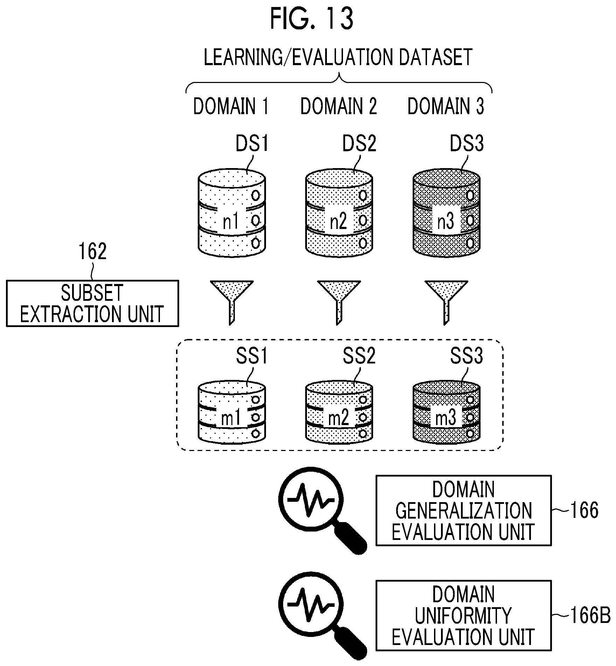

Fig. 13 is an explanatory diagram showing processing according to Embodiment 7. The domain generalization evaluation unit 166 comprises a domain uniformity evaluation unit 166B. The domain uniformity evaluation unit 166B evaluates the domain uniformity indicating the closeness of the distribution between the number of data in the dataset for each domain and the number of data in the subset.

As shown in Fig. 13, here, a dataset DS1 of a domain 1, a dataset DS2 of a domain 2, and a dataset DS3 of a domain 3 are provided as a learning/evaluation dataset. The number of data items of the datasets DS1, DS2, and DS3 is n1, n2, and n3, respectively.

The subset extraction unit 162 extracts subsets SS1, SS2, and SS3 from the datasets DS1, DS2, and DS3 under the designated condition, respectively. The number of data items of the subsets SS1, SS2, and SS3 is m1, m2, and m3, respectively.

The domain generalization evaluation unit 166 evaluates the domain generalization of the subsets SS1, SS2, and SS3.

In addition, the domain uniformity evaluation unit 166B evaluates the domain uniformity. The domain uniformity evaluation unit 166B evaluates that the domain is uniform in a case where the presence ratio of the subsets in the dataset is 40% in all of the domains 1, 2, and 3. In a case where the presence ratio of the subset in the dataset is 90% in the domain 1, 50% in the domain 2, and 10% in the domain 3, the domain uniformity evaluation unit 166B evaluates that the domain is not uniform.

Here, the domain uniformity evaluation unit 166B evaluates the closeness of the distributions by using the numbers n1, n2, and n3 of the data of the datasets DS1, DS2, and DS3 and the numbers m1, m2, and m3 of the data of the subsets SS1, SS2, and SS3. For example, the domain uniformity evaluation unit 166B evaluates that the smaller the Kullback-Leibler (KL) divergence is, the better the domain uniformity is, relatively. The KL divergence can be represented as follows.

$$\text{Kullback-Leibler divergence} = \Sigma_k\, P_1(d = k)\, (\log(P_1(d = k) - \log(P_2(d = k)))$$

k is a domain ID, which is 1, 2, and 3 here. $P_1$ is a distribution of n1, n2, and n3, and $P_1(d = k) = n1/(n1 + n2 + n3)$. In addition, $P_2$ is a distribution of m1, m2, and m3, and $P_2(d = k) = m1/(m1 + m2 + m3)$.

[Embodiment 8]

The information processing apparatus 100 may train the subset classification model. The information processing apparatus 100 may evaluate the subset classification performance of the subset classification model and evaluate the subset by combining the subset classification performance and the domain generalization.

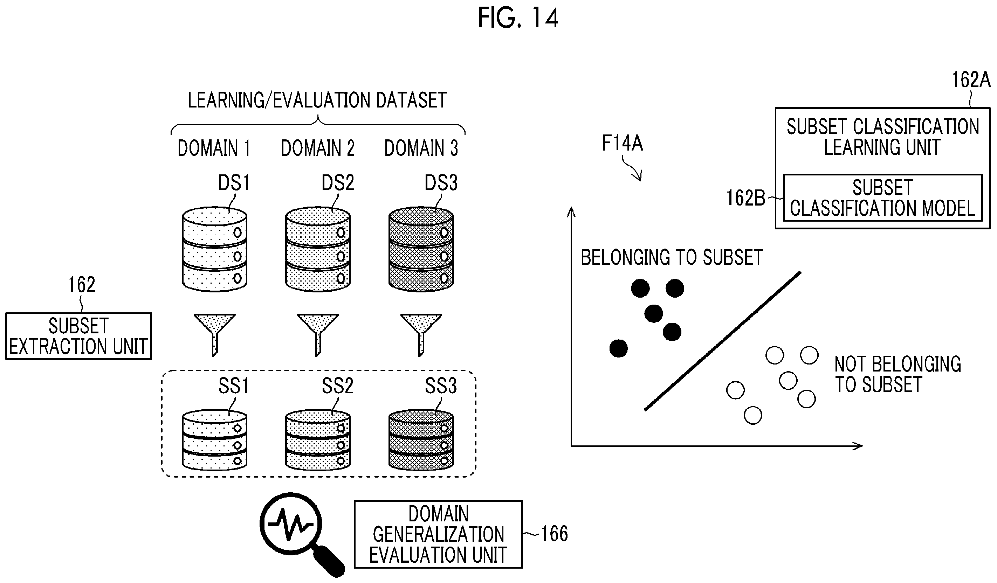

Fig. 14 is an explanatory diagram showing processing according to Embodiment 8. Here, the dataset DS1 of the domain 1, the dataset DS2 of the domain 2, and the dataset DS3 of the domain 3 are provided as the learning/evaluation dataset. The subset extraction unit 162 extracts the subsets SS1, SS2, and SS3 from the datasets DS1, DS2, and DS3, respectively, based on the subset classification model 162B.

Here, the subset extraction unit 162 according to Embodiment 8 comprises a subset classification learning unit 162A. The subset classification learning unit 162A trains a subset classification model 162B that classifies whether or not the data of the dataset is a subset.

For example, it is assumed that the prediction of antibody production uses gene expression as a feature amount and the subset extraction is performed based on DNA mutation. Since it is more convenient to perform the subset extraction by gene expression at the time of operation, a subset classification model from the gene expression is also separately trained. As a matter of course, since it is preferable that the performance of the classification model is better, the ease of classification is also added to the evaluation of the domain generalization of the subset.

F14A of Fig. 14 is a diagram showing a subset extracted from a certain dataset. In F14A, the vertical axis and the horizontal axis are feature amounts or indices calculated from the feature amounts, and the samples of the dataset are plotted according to the values of the vertical axis and the horizontal axis. In this example, an example in which samples of the dataset are classified into samples belonging to a subset indicated by a black circle and samples not belonging to the subset indicated by a white circle is shown. For example, in the VOC classification, a sample belonging to the subset is a user of "cat breeding", and a sample not belonging to the subset is a user other than "cat breeding".

Whether or not the data is a subset is defined by the subset extraction unit 162. F14A shows an example of a case where "cat breeding" is the subset extraction condition. In the example of the stability prediction described later, the antibody production amount being equal to or greater than the average value is the subset extraction condition, and the antibody production amount being equal to or greater than the average value is defined as the subset.

The subset classification learning unit 162A trains the subset classification model 162B by using the data shown in F14A.

The domain generalization evaluation unit 166 evaluates the domain generalization of the subsets SS1, SS2, and SS3. In addition, the domain generalization evaluation unit 166 evaluates the subset classification performance of the subset classification model 162B, and evaluates the subset by combining the subset classification performance and the domain generalization.

In the subset classification performance, as in the evaluation of a general machine learning model, the data is divided into "learning" and "evaluation", and a model trained with the training data is evaluated with the evaluation data. For example, 80% of "cat breeding" and other than "cat breeding" are divided into training data and 20% of "cat breeding" and other than "cat breeding" are divided into evaluation data, and the model trained in the former case is evaluates as to whether or not the model can correctly classify the latter data into "cat breeding" and other than "cat breeding". For the learning and evaluation of the subset classification, data obtained by combining the data of the domain 1, the domain 2, and the domain 3 may be used.

[Embodiment 9]

The information processing apparatus 100 may search for a subset having higher domain generalization by repeating an operation of adding or deleting a sample on a subset from a subset serving as starting point (an example of a "designated condition"). The information processing apparatus 100 may search for the subset by further evaluating any one of usefulness of a subset evaluated from known usefulness information for each sample in the dataset and a sample included in the subset, domain uniformity indicating a closeness in distribution between a number of data items in the dataset and a number of data items in the subset for each of the domains, and a subset classification performance of a subset classification model that classifies whether or not data of the dataset is a subset.

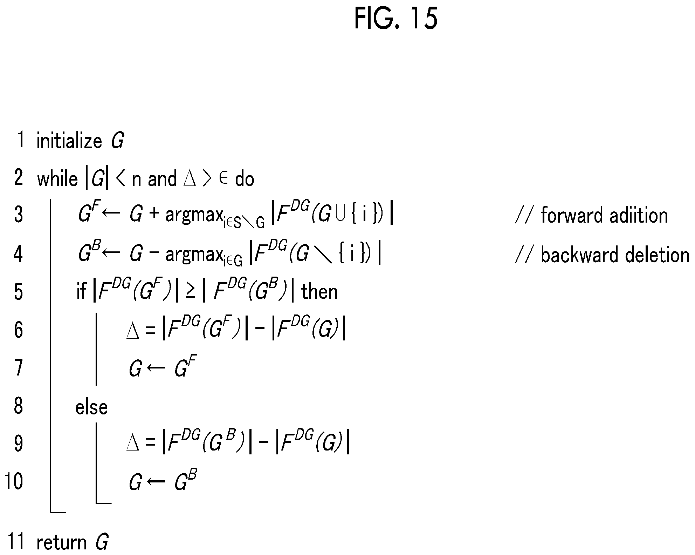

Fig. 15 is an explanatory diagram showing an algorithm of the extraction of the subset according to Embodiment 9. In the present algorithm, the input is $\theta$, which is a threshold value of the degree of association between the feature amount and the response variable, n, which is the maximum size of the subset, and e, which is a convergence condition for ending the improvement of the domain generalization to be less than a certain value, and the output is the subset G having the domain generalization. Here, the domain generalization of the subset is evaluated by the number of feature amounts having domain generalization.

In the first row of Fig. 15, G, which is a subset, is initialized as a subset serving as a starting point. A method of determining the subset serving as a starting point is optional, and the subset may be the entire dataset or an empty set.

In the second row of Fig. 15, $|G|$ representing the number of samples of the subset G for performing the processing and a range of the increase amount in domain generalization $\Delta$ are defined. That is, the processing continues in a case where $|G| < n$ and $\Delta > e$ are satisfied, and the processing ends in a case where the conditions are not satisfied.

In the third row of Fig. 15, a subset $G^F$ obtained by adding one sample from the subset G is defined, and in the fourth row of Fig. 15, a subset $G^B$ obtained by deleting one sample from the subset G is defined. Here, although the number of samples is increased or decreased one by one, a plurality of samples may be increased or decreased together. The target of the argmax may be a weighted sum of not only $|F^{DG}|$ but also the usefulness evaluation, the domain uniformity, and the subset classification performance.

In the fifth row of Fig. 15, it is determined whether or not the subset $G^F$ has relatively higher domain generalization than the subset $G^B$ (whether or not the number of feature amounts having domain generality is large). In a case where the subset $G^F$ has relatively high domain generality, $\Delta$ is updated to $|F^{DG}(G^F)| - |F^{DG}(G)|$ in the sixth row of Fig. 15, and G is updated to $G^F$.

In a case where the domain generality of the subset $G^F$ is not relatively high, $\Delta$ is updated to $|F^{DG}(G^B)| - [F^{DG}(G)|$ in the 9th row of Fig. 15, and G is updated to $G^B$.

The above processing is performed until $|G|$ or $\Delta$ is out of the range defined in the second row of Fig. 15. As a result, it is possible to search for a subset having high domain generalization.

[Embodiment 10]

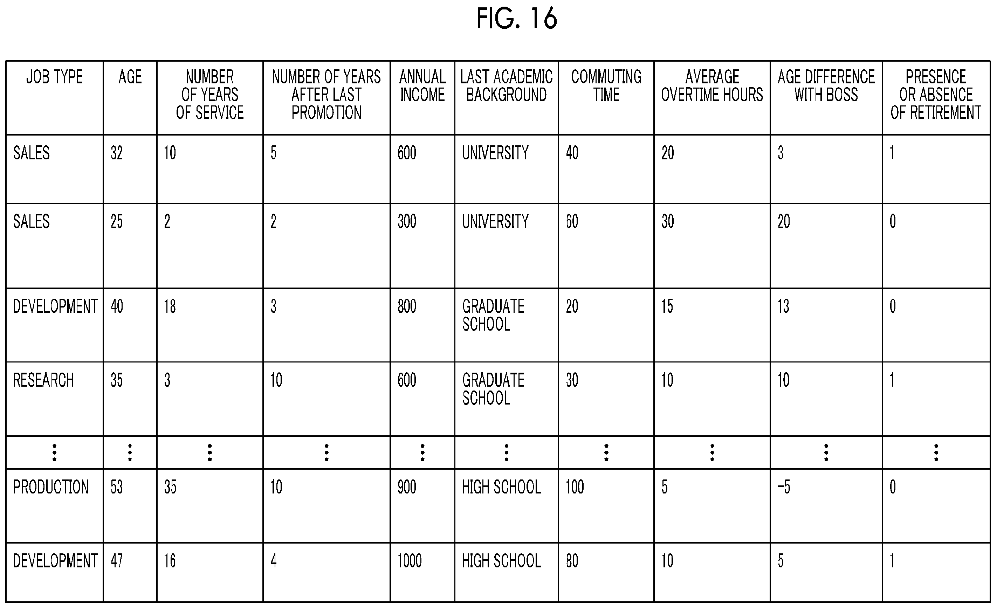

Fig. 16 is a table showing an example of a dataset. The dataset shown in Fig. 16 is a dataset in "Company 1" which is a certain company. As shown in Fig. 16, the dataset has items of "job type", "age", "number of years of service", "number of years after last promotion", "annual income", "last academic background", "commuting time", "average overtime hours", and "age difference with boss" as explanatory variables, and has an item of "presence or absence of retirement" as a response variable for each sample (employee). In addition, "presence or absence of retirement" is "1" for "Yes" and "0" for "No".

For example, in the uppermost row of Fig. 16, the employee has "job type", "age", "number of years of service", "number of years after last promotion", "annual income", "last academic background", "commuting time", "average overtime hours", "age difference with boss", and "presence or absence of retirement" of "sales", "32", "10", "5", "600", "university", "40", "20", "3", and "1", respectively.

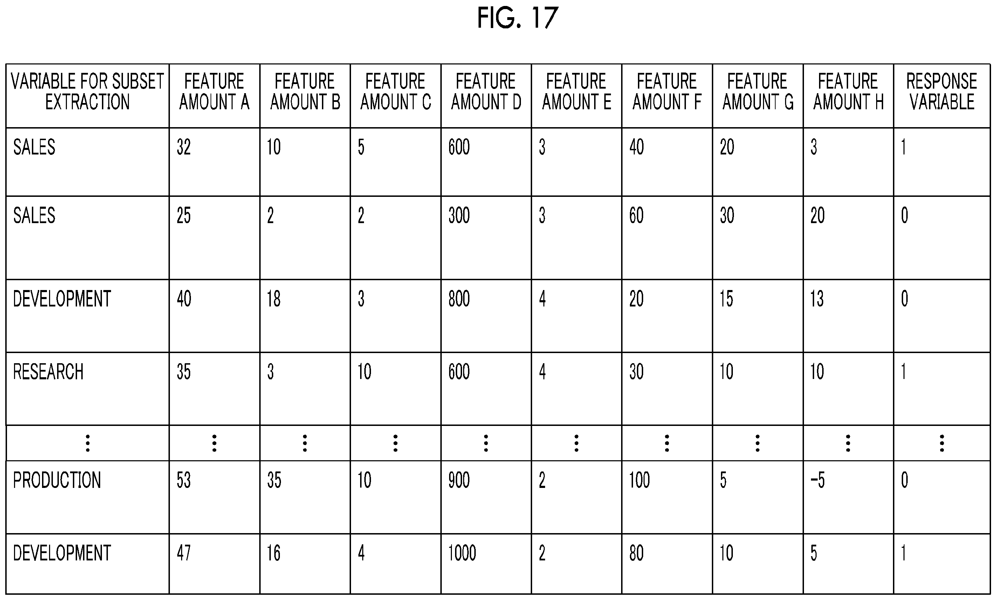

Fig. 17 is a table showing an example of a dataset in which the dataset shown in Fig. 16 is abstracted. Here, the "job type" is "variable for subset extraction", and "age", "number of years of service", "number of years after last promotion", "annual income", "last academic background", "commuting time", "average overtime hours", and "age difference with boss" are "feature amount A", "feature amount B", "feature amount C", "feature amount D", "feature amount E", "feature amount F", "feature amount G", and "feature amount H", respectively. In addition, the "presence or absence of retirement" is "response variable".

The retirement risk prediction model will be described. Here, each company is a domain. There are five datasets of "Company 1", "Company 2", "Company 3", "Company 4", and "Company 5", and it is desired to construct a robust prediction model even for companies (other domains) other than "Company 1" to "Company 5". A prediction model that predicts the presence or absence of retirement (1/0) is constructed based on the eight feature amounts of the feature amounts A to H shown in Fig. 17. The feature amounts are converted into numerical values and all are treated as continuous variables. Here, the subset is extracted based on the "job type". In the extraction of the subset, the feature amount is not narrowed down.

The degree of association between each feature amount and the response variable is evaluated in each of five companies (domains), and the feature amount having a high degree of association in four or more domains out of the five domains is set as the feature amount having high domain generality. The degree of association is evaluated based on the AUC, and in a case where the AUC is 0.8 or more, the degree of association is evaluated as high. More specifically, since the AUC is symmetric around 0.5, it is evaluated that the degree of association is high in a case where |AUC - 0.5| is 0.3 or more. The domain generalization evaluation of the extracted subset is performed based on the number of feature amounts having high domain generality.

First, in a case where the domain generality is evaluated with all the data without extracting the subset, only one feature amount having high domain generality is obtained. Next, a subset is extracted for each job type to evaluate the domain generality. In a case where subsets were extracted for each of "sales", "development", "research", and "production", the number of feature amounts having high domain generality was 5, 2, 1, and 2, respectively. From this, it can be seen that the generality is high in a case where "sales" is extracted as a subset.

Therefore, data of a subset in which only the sales are extracted from the data of each company is prepared, and the retirement risk prediction model is trained using the data. The learned model is expected to have high domain generalization for the subset of "sales", and can be safely used for other unknown companies.

[Other Examples of Dataset]

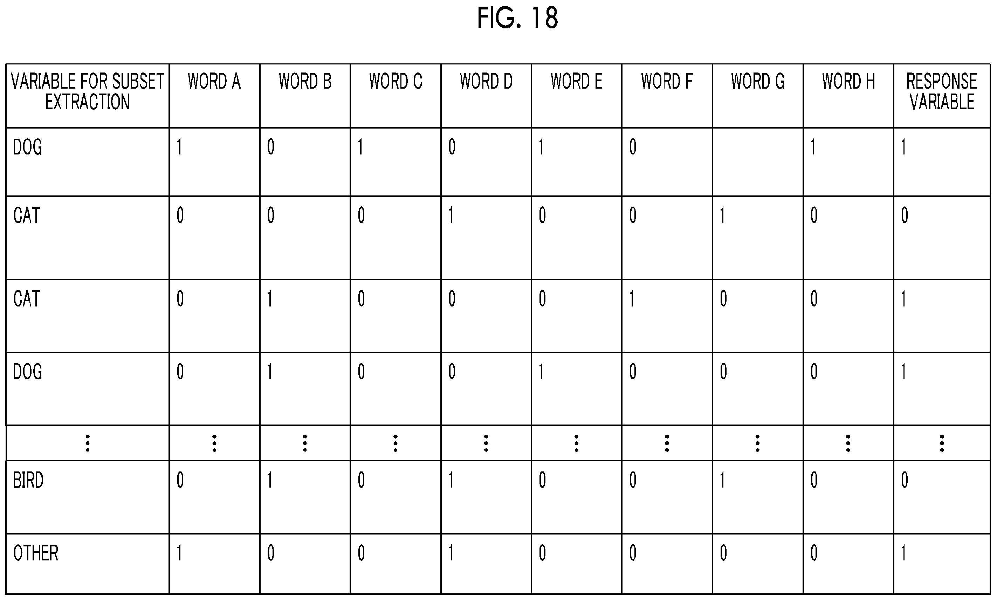

Fig. 18 is a table showing another example of the abstracted dataset. The dataset shown in Fig. 18 is a dataset in a certain company, and it is assumed that a VOC classification model constructed from this dataset is applied to another company.

The dataset shown in Fig. 18 has items of "word A", "word B", "word C", "word D", "word E", "word F", "word G", "word H", and "response variable" in addition to the "variable for subset extraction". The "word A", the "word B", the "word C", the "word D", the "word E", the "word F", the "word G", and the "word H" correspond to the feature amounts, respectively.

[Embodiment 11]

It is widely performed to produce a pharmaceutically useful antibody using Chinese hamster ovary (CHO) cells or the like. In such a production method, it is desirable that the production amount per unit time of the cells is large and the production is stable for a long period of time (the production amount does not change significantly).

Therefore, a model that predicts a stability label (1 or 0) based on the value of the gene expression is constructed. It is desired to construct a model that can predict the stability regardless of the antibody species produced by the cell. That is, the prediction performance for an unknown antibody species is important, and a prediction model having domain generalization with the antibody species as a domain is required.

Here, there is a dataset of five domains of antibody species a, b, c, d, and e. The value of gene expression is a count value that takes a positive integer, and is used as a feature amount after logarithmic conversion. The degree of association between each feature amount and the response variable (stability label) is evaluated for each of five antibody species (domains), and the feature amount having a high degree of association in four or more domains (m = 4) out of five domains is defined as a feature amount having high domain generality. The degree of association is evaluated based on the absolute value of the difference in the response variable of the feature amount average value, and in a case where the difference is 0.2 ($\theta$ = 0.2) or more, it is evaluated that the degree of association is high. The domain generalization evaluation of the extracted subset is performed based on the number |F(DG)| of the feature amounts having high domain generality.

First, in a case where the domain generality is evaluated with all the data without extracting the subset, only one feature amount having high domain generality is obtained. Next, in a case where data in which the antibody production amount was equal to or greater than the average value was extracted as a subset and feature amounts having high domain generality were extracted, the number of feature amounts having high domain generality was 224. Furthermore, in a case where the data in which the viable cell density was equal to or greater than the average value was extracted as a subset, the number of feature amounts having high domain generality was 33. Therefore, the subset is extracted based on the antibody production amount.

A stability prediction model is subjected to machine learning using the data of the extracted subset (the antibody production amount is equal to or more than the average value). Here, a logistic regression model is used. 50 feature amounts are sequentially selected and subjected to training such that the prediction accuracy of the logistic regression is maximized from among 224 feature amounts having high domain generality. Since the training is performed by narrowing down to a subset having high domain generalization, the trained model can predict the stability with robustness even for other antibody species for the subset.

In a case where the stability prediction is operated with the trained model (prediction is made for an untrained antibody species), first, a subset is extracted from samples desired to be predicted under the same conditions (the antibody production amount is equal to or more than the average value), and the stability label is predicted from the gene expression level for the subset. During operation, a sample predicted to be stable is sent to the next step.

In the above example, the domain generalization of the subset is evaluated based on the number of feature amounts having high domain generalization, but the evaluation may be performed based on the ratio of the extracted feature amount that is also effective in another domain.

Specifically, for example, data of four domains of the five domains is used to extract the top 100 feature amounts having a high AUC, and it is evaluated whether or not the feature amounts are also effective for prediction in the remaining one domain (here, a condition that the AUC is 0.6 or more is set). In a case where the number of subsets extracted at the viable cell density is 52 (0.52 as a proportion) and the number of subsets in which the antibody production amount is high is 83 (0.83 as a proportion), it is evaluated that the subset with a high antibody production amount has a higher domain generalization.

In addition, as another evaluation method, as described in Embodiment 2, the domain generalization of the subset may be evaluated by evaluating the prediction performance of the model trained in the four domains in the remaining one domain.

[Regarding Program that Operates Computer]

It is possible to record a program causing a computer to implement some or all of the processing functions of the information processing apparatus 100, in a computer-readable medium that is a non-transitory information storage medium such as an optical disk, a magnetic disk, a semiconductor memory, or other tangible object, and provide the program through this information storage medium.

Also, instead of the aspect in which the program is stored in such a non-transitory computer-readable medium such as tangible object and provided, a program signal can be provided as a download service by using an electric telecommunication line, such as the Internet.

Further, some or all of the processing functions in the information processing apparatus 100 may be implemented by cloud computing or may be provided as a software as a service (SaaS).

[Regarding Hardware Configuration of Each Processing Unit]

A hardware structure of a processing unit that executes various types of processing, such as the dataset acquisition unit 160, the subset extraction unit 162, the learning unit 164, and the domain generalization evaluation unit 166 in the information processing apparatus 100, is, for example, various processors as shown below.

Various processors include a CPU, which is a general-purpose processor that executes a program and functions as various processing units, GPU, a programmable logic device (PLD), which is a processor whose circuit configuration is able to be changed after manufacturing such as a field programmable gate array (FPGA), a dedicated electric circuit, which is a processor having a circuit configuration specially designed to execute specific processing such as an application specific integrated circuit (ASIC), and the like.

One processing unit may be composed of one of the various types of processors or may be composed of two or more processors of the same type or different types. For example, one processing unit may be configured with a plurality of FPGAs, a combination of CPU and FPGA, or a combination of CPU and GPU. Further, a plurality of processing units may be composed of one processor. As an example of configuring a plurality of processing units with one processor, first, as represented by a computer such as a client and a server, there is a form in which one processor is configured by a combination of one or more CPUs and software, and this processor functions as a plurality of processing units. Second, as represented by a system on chip (SoC) or the like, there is a form in which a processor, which implements the functions of the entire system including a plurality of processing units with one integrated circuit (IC) chip, is used. As described above, the various types of processing units are composed of one or more of the various types of processors used as a hardware structure.

Further, as the hardware structure of the various processors, more specifically, an electric circuit (circuitry) in which circuit elements such as semiconductor elements are combined is used.

[Advantages of Embodiment]

According to the present embodiment, it is possible to extract a subset having domain generalization, so that it is possible to construct a classification/prediction model having domain generalization.

[Other Application Examples]

In the above-described embodiment, the model that predicts the risk of employee retirement, the model that predicts the antibody production amount of cells, and the model that classifies the VOCs have been described as examples, but the technology of the present disclosure can be applied to the construction of various classification/prediction models.

The domain generalization evaluation unit 166 may evaluate the domain generalization of the subset by combining the evaluation methods of the subset according to each embodiment.

[Others]

The present disclosure is not limited to the above-described embodiment, and various modifications can be made without departing from the spirit of the idea of the present disclosed technology.

Explanation of References

14: model
100: information processing apparatus
102: processor
104: computer-readable medium
106: communication interface
108: input/output interface
110: bus
112: memory
114: storage
130: extraction program
132: learning program
134: evaluation program
140: dataset storage unit
142: learning model storage unit
152: input device
154: display device
160: dataset acquisition unit

162: subset extraction unit
162A: subset classification learning unit
162B: subset classification model
164: learning unit
166: domain generalization evaluation unit
166A: subset usefulness evaluation unit
166B: domain uniformity evaluation unit
168: subset condition presentation unit
DS1: dataset
DS2: dataset
DS3: dataset
DS4: dataset
DS5: dataset
ED: first evaluation data
ED1: evaluation data
ED2: evaluation data
SS1: subset
SS1A: subset
SS1B: subset
SS2: subset
SS2A: subset
SS2B: subset
SS3: subset
SS3A: subset
SS3B: subset
SS4: subset
SS5: subset
TD: training data
TD1: training data
TD2: training data

**Claims**

1. An information processing method executed by one or more processors, the method comprising:

   via the one or more processors,
   extracting a subset from a dataset to be analyzed under a designated condition; and
   evaluating domain generalization of the extracted subset.

2. The information processing method according to claim **1,**

   wherein the dataset includes datasets of a plurality of domains, and
   the evaluation includes,

      via the one or more processors,
      extracting a learning subset from a dataset of one or more learning domains among the plurality of domains
      under the designated condition and training a learning model by using the learning subset,
      extracting an evaluation subset from a dataset of one or more evaluation domains different from the learning
      domain among the plurality of domains under the designated condition and evaluating the learning model by
      using the evaluation subset, and
      evaluating the domain generalization by using an evaluation result of the learning model.

3. The information processing method according to claim 1,

   wherein the dataset includes datasets of a plurality of domains, and
   the evaluation includes,

via the one or more processors,
extracting a learning subset from a dataset of one or more learning domains among the plurality of domains under the designated condition and training a learning model by using the learning subset,
extracting a first evaluation subset different from the learning subset from the dataset of the learning domain under the designated condition, and evaluating the learning model by using the first evaluation subset,
extracting a second evaluation subset from a dataset of one or more evaluation domains different from the learning domain among the plurality of domains under the designated condition, and evaluating the learning model by using the second evaluation subset, and
evaluating the domain generalization by using a difference between an evaluation result of the first evaluation subset and an evaluation result of the second evaluation subset.

4. The information processing method according to claim 1,

wherein the dataset includes datasets of a plurality of domains, and
the evaluation includes,

via the one or more processors,
evaluating a degree of association between a feature amount of a subset and a response variable for each of the domains, and
assuming that the more the degree of association is relatively high in many domains, the more a domain generality of the feature amount is relatively high, and evaluating that the subset having a larger number of the feature amounts with the relatively high domain generality has a relatively high domain generalization.

5. The information processing method according to claim 1,

wherein the dataset includes datasets of a plurality of domains, and
the evaluation includes,

via the one or more processors,
evaluating a degree of association between a feature amount of a subset and a response variable for each of the domains, and
setting a feature amount having a degree of association equal to or greater than a threshold value in a certain number or more of domains as a feature amount having relatively high domain generality, and evaluating the domain generalization of the subset by using the number of the feature amounts having the relatively high domain generality.

6. The information processing method according to claim 1,

wherein the dataset includes datasets of a plurality of domains, and
the evaluation includes,

via the one or more processors,
extracting a learning subset from a dataset of one or more learning domains among the plurality of domains under the designated condition and extracting a feature amount set from the learning subset,
extracting an evaluation subset from a dataset of one or more evaluation domains different from the learning domain among the plurality of domains under the designated condition, and evaluating the extracted feature amount set by using the evaluation subset, and
evaluating the domain generalization of the learning subset by using a proportion of a feature amount that is effective in the evaluation subset among feature amounts of the extracted feature amount set.

7. The information processing method according to claim 6, further comprising:

via the one or more processors,
evaluating a degree of association between a feature amount of the learning subset and a response variable for each of the domains; and
assuming that the more the degree of association is relatively high in many domains, the more a domain generality of the feature amount is relatively high,
wherein the extracted feature amount set includes the feature amount having the relatively high domain generality.

8. The information processing method according to claim 6, further comprising:

via the one or more processors,
evaluating a degree of association between a feature amount of the learning subset and a response variable for each of the domains; and
setting a feature amount having a degree of association equal to or greater than a threshold value in a certain number or more of domains as a feature amount having relatively high domain generality,
wherein the extracted feature amount set includes the feature amount having the relatively high domain generality.

9. The information processing method according to claim 1,
wherein the evaluation includes,

via the one or more processors,
evaluating usefulness of the subset from known usefulness information for each sample in the dataset and a sample included in the subset, and
evaluating a subset by combining the usefulness and the domain generalization.

10. The information processing method according to claim 1,

wherein the dataset includes datasets of a plurality of domains, and
the evaluation includes,

via the one or more processors,
evaluating domain uniformity indicating a closeness in distribution between a number of data items in the dataset and a number of data items in the subset for each of the domains, and
evaluating the subset by combining the domain uniformity and the domain generalization.

11. The information processing method according to claim 1, further comprising:

via the one or more processors,
training a subset classification model that classifies whether or not data of the dataset is a subset.

12. The information processing method according to claim 11,
wherein the evaluation includes,

via the one or more processors,
evaluating a subset classification performance of the subset classification model, and
evaluating the subset by combining the subset classification performance and the domain generalization.

13. The information processing method according to claim 1,
wherein the extraction and the evaluation include,

via the one or more processors,
searching for a subset having a higher domain generalization by repeating an operation of adding or deleting a sample on the subset, from a subset serving as a starting point.

14. The information processing method according to claim 13,

wherein the dataset includes datasets of a plurality of domains, and
the searching includes,

via the one or more processors,
searching for the subset by further evaluating any one of usefulness of a subset evaluated from known usefulness information for each sample in the dataset and a sample included in the subset, domain uniformity indicating a closeness in distribution between a number of data items in the dataset and a number of data items in the subset for each of the domains, and a subset classification performance of a subset classification model that classifies whether or not data of the dataset is a subset.

15. The information processing method according to any one of claims 1 to 14, further comprising:

   via the one or more processors,
   presenting a plurality of different subset conditions;
   evaluating subsets extracted under each of the plurality of different subset conditions; and
   extracting a subset under a subset condition having a best evaluation result among the plurality of different subset conditions.

16. An information processing apparatus comprising:

   one or more processors; and
   one or more memories in which a command to be executed by the one or more processors is stored,
   wherein the one or more processors are configured to:

      extract a subset of a dataset to be analyzed under a designated condition; and
      evaluate domain generalization of the extracted subset.

17. A program causing a computer to implement:

   a function of extracting a subset of a dataset to be analyzed under a designated condition; and
   a function of evaluating domain generalization of the extracted subset.

18. A non-transitory computer-readable recording medium on which the program according to claim 17 is recorded.

# FIG. 1

COLLECT DATA
AT CERTAIN FACILITY

$P_{d1}(X,Y)$

Step 1: TRAIN MODEL

MODEL
14

INTRODUCE MODEL
INTO DIFFERENT FACILITY

$P_{d2}(X,Y)$

Step 2: INTRODUCE MODEL
14

MODEL

EP 4 583 018 A1

# FIG. 2

INTRODUCTION DESTINATION
FACILITY AND USER

DATA COLLECTED
AT DIFFERENT FACILITY

DATA COLLECTED
AT INTRODUCTION
DESTINATION FACILITY

Step 1: TRAIN MODEL

14

MODEL

Step 2: OPERATE MODEL

14

MODEL

EP 4 583 018 A1

## FIG. 3

TRAINING DATA  EVALUATION DATA 1
(SAME DOMAIN)

EVALUATION DATA 2
(DIFFERENT DOMAIN)

DATA IS GENERATED BY $P_{d1}(X, Y)$     DATA IS GENERATED BY $P_{d2}(X, Y)$

## FIG. 4

PERFORMANCE A:
PERFORMANCE IN
TRAINING DATA

PERFORMANCE B:
PERFORMANCE IN
EVALUATION DATA 1
(SAME DATA)

PERFORMANCE C:
PERFORMANCE IN
EVALUATION DATA 2
(DIFFERENT DATA)

## FIG. 5

**100**

**COMPUTER-READABLE MEDIUM** ~104

**102 PROCESSOR**

**110**

**106 COMMUNICATION INTERFACE**

**108 INPUT/OUTPUT INTERFACE**

**112 MEMORY**

130~ **SUBSET EXTRACTION PROGRAM** ← **DATASET STORAGE UNIT** ~140

132~ **LEARNING PROGRAM** ↔ **LEARNING MODEL STORAGE UNIT** ~142

134~ **DOMAIN GENERALIZATION EVALUATION PROGRAM**

**114 STORAGE**

152~ **INPUT DEVICE** **DISPLAY DEVICE** ~154

FIG. 6

100

160
DATASET
ACQUISITION
UNIT

162
SUBSET
EXTRACTION
UNIT

164
LEARNING UNIT

166
DOMAIN
GENERALIZATION
EVALUATION UNIT

140
DATASET
STORAGE UNIT

142
LEARNING
MODEL
STORAGE UNIT

FIG. 7

# FIG. 8

LEARNING DOMAIN

EVALUATION DOMAIN

DOMAIN 1　　DOMAIN 2　　DOMAIN 3

DS1　　DS2　　DS3

SS1　　SS2　　SS3

EVALUATION DATA 2

TD1

ED1

TD2

ED2

TD

ED

TRAINING DATA

EVALUATION DATA 1

# FIG. 9

LEARNING DOMAIN

EVALUATION DOMAIN

DOMAIN 1    DOMAIN 2    DOMAIN 3

DS1    DS2    DS3

SS1    SS2    SS3

VS

EXTRACTED FEATURE AMOUNT SET

VS

VERIFY EFFECTIVENESS IN EVALUATION DOMAIN

VS   F9A

PROPORTION OF FEATURE AMOUNT THAT IS ALSO EFFECTIVE IN EVALUATION DOMAIN (FILLED IN AREA)

# FIG. 10

LEARNING/EVALUATION DATASET

| DOMAIN 1 | DOMAIN 2 | DOMAIN 3 |
|----------|----------|----------|
| DS1 | DS2 | DS3 |

168
SUBSET CONDITION
PRESENTATION UNIT

SUBSET CONDITION A

SUBSET CONDITION B

162
SUBSET
EXTRACTION UNIT

SS1A    SS2A    SS3A

SUBSET CONDITION A

166
DOMAIN
GENERALIZATION
EVALUATION UNIT

162
SUBSET
EXTRACTION UNIT

SS1B    SS2B    SS3B

SUBSET CONDITION B

ADOPT A IN CASE WHERE
EVALUATION RESULT IS A > B

166
DOMAIN
GENERALIZATION
EVALUATION UNIT

EP 4 583 018 A1

# FIG. 11

LEARNING/EVALUATION DATASET

DOMAIN 1    DOMAIN 2    DOMAIN 3

DS1    DS2    DS3

162
SUBSET
EXTRACTION UNIT

SS1    SS2    SS3

DOMAIN
GENERALIZATION
EVALUATION UNIT ~166

SUBSET
USEFULNESS
EVALUATION UNIT ~166A

# FIG. 12

NUMBER OF ANNUAL
PRINTED PHOTOGRAPHS
(AVERAGE)

CAT BREEDING    DOG BREEDING    OTHER PET    NO PET

# FIG. 13

LEARNING/EVALUATION DATASET

DOMAIN 1     DOMAIN 2     DOMAIN 3

DS1     DS2     DS3

n1     n2     n3

162

SUBSET
EXTRACTION UNIT

SS1     SS2     SS3

m1     m2     m3

DOMAIN
GENERALIZATION
EVALUATION UNIT  ~166

DOMAIN
UNIFORMITY
EVALUATION UNIT  ~166B

# FIG. 14

LEARNING/EVALUATION DATASET

DOMAIN 1    DOMAIN 2    DOMAIN 3

DS1      DS2      DS3

162
SUBSET EXTRACTION UNIT

SS1      SS2      SS3

DOMAIN GENERALIZATION EVALUATION UNIT ~166

F14A

162A
SUBSET CLASSIFICATION LEARNING UNIT

162B— SUBSET CLASSIFICATION MODEL

BELONGING TO SUBSET

NOT BELONGING TO SUBSET

## FIG. 15

initialize $G$

while $|G| < n$ and $\triangle > \epsilon$ do

$\quad G^F \leftarrow G + \mathrm{argmax}_{i \in S \setminus G} |F^{DG}(G \cup \{i\})|$      // forward adiition

$\quad G^B \leftarrow G - \mathrm{argmax}_{i \in G} |F^{DG}(G \setminus \{i\})|$      // backward deletion

$\quad$ if $|F^{DG}(G^F)| \geq |F^{DG}(G^B)|$ then

$\quad\quad \triangle = |F^{DG}(G^F)| - |F^{DG}(G)|$

$\quad\quad G \leftarrow G^F$

$\quad$ else

$\quad\quad \triangle = |F^{DG}(G^B)| - |F^{DG}(G)|$

$\quad\quad G \leftarrow G^B$

return $G$

## FIG. 16

| JOB TYPE | AGE | NUMBER OF YEARS OF SERVICE | NUMBER OF YEARS AFTER LAST PROMOTION | ANNUAL INCOME | LAST ACADEMIC BACKGROUND | COMMUTING TIME | AVERAGE OVERTIME HOURS | AGE DIFFERENCE WITH BOSS | PRESENCE OR ABSENCE OF RETIREMENT |
|---|---|---|---|---|---|---|---|---|---|
| SALES | 32 | 10 | 5 | 600 | UNIVERSITY | 40 | 20 | 3 | 1 |
| SALES | 25 | 2 | 2 | 300 | UNIVERSITY | 60 | 30 | 20 | 0 |
| DEVELOPMENT | 40 | 18 | 3 | 800 | GRADUATE SCHOOL | 20 | 15 | 13 | 0 |
| RESEARCH | 35 | 3 | 10 | 600 | GRADUATE SCHOOL | 30 | 10 | 10 | 1 |
| ⋮ | ⋮ | ⋮ | ⋮ | ⋮ | ⋮ | ⋮ | ⋮ | ⋮ | ⋮ |
| PRODUCTION | 53 | 35 | 10 | 900 | HIGH SCHOOL | 100 | 5 | −5 | 0 |
| DEVELOPMENT | 47 | 16 | 4 | 1000 | HIGH SCHOOL | 80 | 10 | 5 | 1 |

EP 4 583 018 A1

## FIG. 17

| VARIABLE FOR SUBSET EXTRACTION | FEATURE AMOUNT A | FEATURE AMOUNT B | FEATURE AMOUNT C | FEATURE AMOUNT D | FEATURE AMOUNT E | FEATURE AMOUNT F | FEATURE AMOUNT G | FEATURE AMOUNT H | RESPONSE VARIABLE |
|---|---|---|---|---|---|---|---|---|---|
| SALES | 32 | 10 | 5 | 600 | 3 | 40 | 20 | 3 | 1 |
| SALES | 25 | 2 | 2 | 300 | 3 | 60 | 30 | 20 | 0 |
| DEVELOPMENT | 40 | 18 | 3 | 800 | 4 | 20 | 15 | 13 | 0 |
| RESEARCH | 35 | 3 | 10 | 600 | 4 | 30 | 10 | 10 | 1 |
| ⋮ | ⋮ | ⋮ | ⋮ | ⋮ | ⋮ | ⋮ | ⋮ | ⋮ | ⋮ |
| PRODUCTION | 53 | 35 | 10 | 900 | 2 | 100 | 5 | −5 | 0 |
| DEVELOPMENT | 47 | 16 | 4 | 1000 | 2 | 80 | 10 | 5 | 1 |

# FIG. 18

| VARIABLE FOR SUBSET EXTRACTION | WORD A | WORD B | WORD C | WORD D | WORD E | WORD F | WORD G | WORD H | RESPONSE VARIABLE |
|---|---|---|---|---|---|---|---|---|---|
| DOG | 1 | 0 | 1 | 0 | 1 | 0 |  | 1 | 1 |
| CAT | 0 | 0 | 0 | 1 | 0 | 0 | 1 | 0 | 0 |
| CAT | 0 | 1 | 0 | 0 | 0 | 1 | 0 | 0 | 1 |
| DOG | 0 | 1 | 0 | 0 | 1 | 0 | 0 | 0 | 1 |
| ⋮ | ⋮ | ⋮ | ⋮ | ⋮ | ⋮ | ⋮ | ⋮ | ⋮ | ⋮ |
| BIRD | 0 | 1 | 0 | 1 | 0 | 0 | 1 | 0 | 0 |
| OTHER | 1 | 0 | 0 | 1 | 0 | 0 | 0 | 0 | 1 |

EP 4 583 018 A1

## INTERNATIONAL SEARCH REPORT

| International application No. |
|---|
| **PCT/JP2023/025262** |

| A. | CLASSIFICATION OF SUBJECT MATTER |
|---|---|

*G06N 20/00*(2019.01)i
FI:   G06N20/00

According to International Patent Classification (IPC) or to both national classification and IPC

| B. | FIELDS SEARCHED |
|---|---|

Minimum documentation searched (classification system followed by classification symbols)

G06N20/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | 芦川 博人 ほか, ドメイン属性情報を用いたRNN言語モデルのドメイン汎化, 日本音響学会 2019年 春季研究発表会講演論文集 [CD-ROM], 02 April 2019 (received date), pp. 927-930, non-official translation (ASHIKAWA, Hiroto et al. Domain Generalization of RNN Language Models Using Domain Attribute Information. Proceedings of the Acoustical Society of Japan 2019 Spring Meeting Japan.)<br>p. 928, right column to p. 929, right column, table 4 | 1-3, 11, 15-18 |
| A | GRAG, Vikas K. et al. Learn to Expect the Unexpected: Probably Approximately Correct Domain Generalization. ArXiv [online]. 13 February 2020, pp. 1-17, [retrieved on 11 September 2023], Internet: <URL: https://arxiv.org/pdf/2002.05660v1.pdf><br>entire text | 1-18 |
| A | US 2017/0220897 A1 (XEROX CORPORATION) 03 August 2017 (2017-08-03)<br>entire text | 1-18 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 September 2023** | **26 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

International application No.

**PCT/JP2023/025262**

| Patent document cited in search report | Publication date (day/month/year) | Patent family member(s) | Publication date (day/month/year) |
|---|---|---|---|
| US 2017/0220897 A1 | 03 August 2017 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VIKAS GARG** ; **ADAM TAUMAN KALAI** ; **KATRINA LIGETT** ; **STEVEN WU**. Learn to Expect the Unexpected: Probably Approximately Correct Domain Generalization. AISTATS, 2021
- **IVAN CANTADOR** ; **IGNACIO FENANDEZ-TO-BIAS** ; **SHLOMO BWRKOVSKY** ; **PAOLO CREMO-NESI**. Cross-domain Recommender System. Springer, 2015

- A survey on feature selection methods. **G CHAN-DRASHEKAR** ; **F SAHIN**. Computers & Electrical Engineering. Elsevier, 2014
- **Y SAEYS** ; **I INZA** ; **P LARRANAGA**. A review of feature selection techniques in bioinformatics. *bioinformatics*, 2007